# EUROPEAN PATENT SPECIFICATION

(11) **EP 4 389 196 B1**
(45) Date of publication and mention of the grant of the patent: **24.06.2026**
(21) Application number: 22214686.2
(22) Date of filing: 19.12.2022
(51) Int. Cl.: A61N 1/36

(54) **SYSTEM AND COMPUTER PROGRAM FOR OPERATING A NEUROSTIMULATION DEVICE WITH LESS SIDE EFFECTS**
SYSTEM UND COMPUTERPROGRAMM ZUM BETREIBEN EINER NEUROSTIMULATIONSVORRICHTUNG MIT WENIGER NEBENWIRKUNGEN
SYSTÈME ET PROGRAMME INFORMATIQUE POUR FAIRE FONCTIONNER UN DISPOSITIF DE NEUROSTIMULATION AVEC MOINS D'EFFETS SECONDAIRES

(43) Date of publication of application: 26.06.2024
(73) Proprietor: CereGate GmbH, 81671 München (DE)
(72) Inventor: HAGH GOOIE, Saman, 81671 München (DE); VÁRKUTI, Bálint, 81679 München (DE); MANOLA, Ljubomir, 81671 München (DE)
(74) Representative: Bardehle Pagenberg Partnerschaft mbB Patentanwälte Rechtsanwälte

(56) References cited:
- WO-A1-2022/183198
- US-A1- 2011 160 796
- US-A1- 2021 346 699
- US-B1- 9 492 667

## Description

### Technical field

The present disclosure relates to a method, system, and non-transitory computer readable memory medium for configuring a neurostimulation device to cause a neurophysiologic effect (e.g., to treat a neurological disorder, operate a computer brain interface, etc.) with reduced side effects and, preferably, enhanced effectiveness / efficacy. The present disclosure further relates to a neurostimulation device that is configured to generate and apply such neurostimulation sequences that cause a desired neurophysiologic effect with reduced side effects.

### Technical background

Neurostimulation devices such as implantable pulse generators (IPGs) can be used to treat a variety of neurological disorders, such as cardiac arrhythmia, cardiac fibrillation, deafness, blindness, limb movement, chronic pain, motor and psychological disorders. Particularly, deep brain stimulation (DBS) can be used for treating neurologic symptoms arising from Parkinson disease, dystonia , and essential tremor and similar neurologic disorders. Typically, such neurostimulation devices generate and deliver neurostimulation pulses to treatment areas within the central nervous system (CNS), i.e., to certain brain regions or parts of the spinal cord that are associated with the disorder to be treated. Other neurostimulation devices may also be configured to stimulate the peripheral nervous system (PNS). For example, PNS stimulation paradigms comprise Vagus nerve stimulation for treatment of epilepsy or depression as well as occipital or trigeminal nerve stimulation for treatment of headaches such as migraine. PNS stimulation is also used for pain management to block pain signals before being relayed to the CNS. Further, some neurostimulation devices may also be configured to provide intelligible input to the nervous system, e.g., to implement a sensory computer brain interface (CBI) like the technologies described in WO2020/174051 A1, titled *NEURONAL COMMUNICATION SYSTEM*.

As an example, essential tremor can be considered which is one of the most common neurological disorders and causes an involuntary, oscillatory movement of one or more body parts, such as the arms or hands. Thereby, tremor impairs quality of life and leads to disability and social handicap. It is estimated that approximate 0.9% of people worldwide are affected by essential tremor. As it is expected that the number of persons with tremor and similar conditions increases, improvements of conventional neurostimulation therapy is also desired.

Typically, for treatment of neurologic disorders, an IPG or similar neurostimulation device provides sequences of neurostimulation pulses to stimulation leads / electrodes implanted in proximity to a specific treatment area in the nervous system of a person. For treating tremor, the stimulation leads are implanted in proximity to the Ventral intermediate nucleus (ViM) of the thalamus or a subthalamic area (STA). For treating symptoms of Parkinson disease, the stimulation electrodes can be implanted in proximity to the subthalamic nucleus (STN).

Conventional neurostimulation therapy devices may cause undesired side effects. These side effects may for instance be caused by the blockage of, or interference with natural brain activity caused by the applied stimulation pulses and thereby may lead to malfunctioning of perception, seizures, headache, confusion or similar side effects. Neurostimulation therapy or CBI operation may also impair normal motoric functions. Such side effects are highly undesired since they may lead to reduced treatment acceptance and even may cause reversible or irreversible damage to the nervous system.

In this context, EP 3 086 839 B1 relates to a programming system for an implantable deep brain stimulator. A medical stimulation system is provided having a clinical programmer configured to operate on a computational and memory device having a wireless communication device. Further, a neurostimulator is provided, which is configured to wirelessly communicate with the clinical programmer. The neurostimulator also includes a pulse generator configured to transmit an electrical signal having pulse trains to treat a neurological condition.

US 7483,747 B2 relates to systems and methods for enhancing or affecting neural stimulation efficiency and/or efficacy are disclosed. In one discussed example, a system and/or method may apply electromagnetic stimulation to a patient's nervous system over a first time domain according to a first set of stimulation parameters, and over a second time domain according to a second set of stimulation parameters. The first and second time domains may be sequential, simultaneous, or nested. Stimulation parameters may vary in accordance with one or more types of duty cycle, amplitude, pulse repetition frequency, pulse width, spatiotemporal, and/or polarity variations. Stimulation may be applied at subthreshold, threshold, and/or suprathreshold levels in one or more periodic, aperiodic (e.g., chaotic), and/or pseudo-random manners. In some discussed examples, stimulation may comprise a burst pattern having an interburst frequency corresponding to an intrinsic brainwave frequency, and regular and/or varying intraburst stimulation parameters. In some discussed examples, stimulation signals providing reduced power consumption with at least adequate symptomatic relief may be applied prior to moderate or significant power source depletion.

US 9,119,964 B2 relates to an implantable stimulator system including a motion sensor. Said sensor monitors person movement and communicates with the implantable stimulator to provide closed loop control based on such movement.

US 2021/0260365 A1 relates to a stimulation system and method which can be utilized to treat neurological conditions and/or disorders. In particular, a neuromodulation design or stimulation parameters are described in which the stimulation parameters produce burst stimulation to override or alter a pathological stimulation to treat a neurological condition.

WO 2022/183198 A1 relates to a system including electrical stimulation circuitry configured to generate electrical stimulation, electrodes configured to deliver the electrical stimulation to a patient, and processing circuitry configured to determine, for a patient, a first cycling of electric stimulation doses.

### Summary

In view of the foregoing, new neurostimulation methods, systems and devices are desired that maintain or even improve a desired neurophysiologic effect (e.g., treatment effectiveness and / or efficacy; CBI performance and / or fidelity and / or bandwidth) while reducing side effects caused by conventional stimulations paradigms. This and similar problems are at least partially solved by the subject-matter of the appended claims.

Specifically, aspects of the present disclosure relate to applying, using a neurostimulation device, a neurostimulation sequence comprising a plurality of stimulation pulses to a nervous system of a person, while determining a neurophysiologic effect strength caused by the neurostimulation sequence, a side effect strength associated with the neurostimulation sequence, or both, and generating, based on determining the neurophysiologic effect strength, the side effect strength, or both, a modified neurostimulation sequence having an increased quiescence time, wherein generating the modified neurostimulation sequence comprises: reducing a number of stimulation pulses of the neurostimulation sequence by including one or more quiescence periods into the neurostimulation sequence, or by removing stimulation pulses from the neurostimulation sequence, or by doing both, and increasing one or more of a pulse frequency, a pulse amplitude or a pulse width of the neurostimulation sequence to increase a charge injected into the nervous system, and storing the modified neurostimulation sequence in a non-transitory computer-readable memory medium. For example, the pulse frequency, pulse amplitude and /or pulse width of at least a portion of the neurostimulation sequence may be increased to at least partially compensate for the decrease in charge injected caused by increasing the quiescence time.

In this manner, effectiveness and / or efficacy of the desired neurophysiologic effect (e.g., effectiveness and / or efficacy of a neurostimulation therapy applied by the configured neurostimulation device) can be maintained or even enhanced while the severity of associated side effects can be reduced, e.g., because the time that the CNS and / or the PNS of the person can operate undisturbed (e.g., without receiving neurostimulation) is increased. For applications in CBI devices and systems aspects of the present disclosure allow to maintain or improve CBI performance, fidelity and / or bandwidth while decreasing side effects caused by using the CBI to communicate / transmit information to the person. Aspects of the present disclosure thus allow to iteratively optimize the positions, durations and / or the (e.g., pseudo-random) distribution of the quiescence periods within a neurostimulation sequence while observing a constraint for the effect strength, the side effect strength or both. In some aspects, a stimulation pulse removal probability may be optimized in the same manner.

For instance, increasing the quiescence time as described above may reduce the number of action potentials elicited by the neurostimulation sequence that may disrupt or adversely affect the normal function of the nervous system of the person. For example, aspects of the present disclosure allow to reduce the number of antidromic action potentials which may annihilate normal physiologic action potentials traveling in the opposite orthodromic direction (e.g., orthodromic action potentials traveling from a sensory organ of the person to a corresponding sensory cortex area or orthodromic action potentials traveling from spinal interneurons up the dorsal column along the afferent pathways into the brain, or cranially, from the cerebellum to thalamus or from thalamus to the motor cortex). In this manner, informational lesions caused by the modified neurostimulation sequence can be reduced while effectiveness and / or efficacy of the desired neurophysiologic effect can be maintained. In some aspects, the quiescence time is increased in a random (e.g., pseudo-random) manner (e.g., by randomly including quiescence periods into a neurostimulation sequence as discussed in detail below) e.g., to counter habituation effects by the brain of the person. Thus, aspects of the present disclosure allow to reduce side effects while effectiveness and / or efficacy of the desired neurophysiologic effect (e.g., treatment of symptoms of a neurologic disorder; communicating information via a (sensory) CBI, etc.) can be maintained or even be increased.

In some aspects, generating and storing the modified neurostimulation sequence may comprise: reducing the number of stimulation pulses of the neurostimulation sequence, and applying, using the neurostimulation device, the modified neurostimulation sequence to the nervous system of the person while determining the neurophysiologic effect strength (e.g., via measuring a physiologic parameter of the person associated with the neurophysiologic effect), determining whether the neurophysiologic effect strength remains above a preconfigured threshold for the neurophysiologic effect strength and / or determining whether the side effect strength remains below a preconfigure threshold while the modified neurostimulation sequence is applied, and, based on the determination whether the effect strength remains above the preconfigured threshold and / or based on the determination whether the side effect strength remains below the preconfigured threshold, further modifying the neurostimulation sequence (as discussed in the preceding paragraphs) or storing the modified neurostimulation sequence in the non-transitory computer-readable memory medium.

Further aspects of the present disclosure relate to a system comprising a neurostimulation device adapted to apply a plurality of neurostimulation sequences, each comprising a plurality of stimulation pulses to a nervous system of a person; and a programming device, operably connected to the neurostimulation device, wherein the programming device and the neurostimulation device are adapted to: apply a neurostimulation sequence comprising a plurality of stimulation pulses to a nervous system of a person, while determining a neurophysiologic effect strength caused by the neurostimulation sequence, a side effect strength associated with the neurostimulation sequence, or both generate, based on the determined neurophysiologic effect strength, the side effect strength, or both, a modified neurostimulation sequence having an increased quiescence time; wherein generating the modified neurostimulation sequence comprises: reducing a number of stimulation pulses of the neurostimulation sequence by including one or more quiescence periods into the neurostimulation sequence, or by randomly removing stimulation pulses from the neurostimulation, or by both; and increasing one or more of a pulse frequency, a pulse amplitude or the pulse width of the neurostimulation sequence to increase a charge injected into the nervous system, and store the modified neurostimulation sequence in a non-transitory computer-readable memory medium of the neurostimulation device. The modified neurostimulation sequence can be obtained from the non-transitory computer-readable memory medium and be used for treating the neurologic disorder with reduced side effects. The programming device can be integrated with the neurostimulation device. Further, the system may also comprise one or more sensors for measuring a physiologic parameter of the person that may be indicative of the neurophysiologic effect strength and / or indicative of the side effect strength. The system may further include devices for assessing how well the person carries out a task that may be affected by the neurophysiologic effect, the side effect or both.

Further aspects of the present disclosure relate to a non-transitory computer-readable memory medium comprising instructions for causing a neurostimulation device and a programming device as described above to carry out the methods described herein.

Further aspects of the present disclosure relate to a neurostimulation device comprising a processor operably connected to neurostimulation circuitry and adapted to generate and apply a plurality of neurostimulation sequences to a nervous system of a person. The device may also comprise a non-transitory computer readable memory, operably connected to the processor and storing a first and one or more second neurostimulation sequences, each sequence comprising a respective plurality of stimulation pulses and being adapted to cause a neurophysiologic effect. The one or more second neurostimulation sequences may comprise a larger quiescence time than the first neurostimulation sequence by having less stimulation pulses and the one or more second neurostimulation sequences may comprise one or more of: an increased pulse frequency, an increased pulse amplitude or an increased pulse width. For instance, a subset or all stimulation pulses of the one or more second neurostimulation sequence may comprise an increased pulse frequency, an increased pulse amplitude and / or an increased pulse width as compared to the first neurostimulation sequence.

Further aspects of the present disclosure and related benefits are described in the following with reference to the appended figures.

### Brief description of the figures

Various aspects of the present disclosure are described in the following by reference to the accompanying figures. These figures show:
- **Fig. 1**: a schematic illustration of a system for configuring a neurostimulation device according to aspects of the present disclosure;
- **Fig. 2**: a schematic illustration showing how an output signal of a sensor measuring a physiologic parameter of a person can be used to determine a neurophysiologic effect strength;
- **Fig. 3**: a schematic block diagram of a closed-loop system for configuring a neurostimulation device according to aspects of the present disclosure;
- **Fig. 4**: a schematic diagram illustrating an iterative procedure for configuring a neurostimulation device according to aspects of the present disclosure;
- **Fig. 5**: a further schematic diagram illustrating an iterative multi-parameter optimization procedure for configuring a neurostimulation device according to aspects of the present disclosure;
- **Fig. 6**: a detailed excerpt of Fig. 5 illustrating further aspects of the optimization procedure disclosed herein;
- **Fig. 7**: illustrates how the quiescence time of an initial neurostimulation sequence can iteratively be increased by including one or more quiescence periods to arrive at a modified neurostimulation sequence according to aspects of the present disclosure;
- **Fig. 8**: illustrates how the quiescence time of an initial neurostimulation sequence can iteratively be increased by randomly or pseudo-randomly removing one or more stimulation pulses according to aspects of the present disclosure;
- **Fig. 9A**: a schematic illustration of a neurostimulation sequence comprising multi-phasic stimulation pulses according to aspects of the present disclosure;
- **Fig. 9B**: a schematic illustration of a multi-phasic stimulation pulse according to aspects of the present disclosure;
- **Fig. 10**: a schematic illustration of a person using a neurostimulation device according to aspects of the present disclosure;
- **Fig. 11**: a schematic illustration of a person using a neurostimulation device according to aspects of the present disclosure;
- **Fig. 12**: a functional block circuit diagram illustrating an exemplary programmer device according to aspects of the present disclosure;
- **Fig. 13**: a functional block circuit diagram illustrating an exemplary neurostimulation device according to aspects of the present disclosure;
- **Fig. 14**: a process diagram illustrating a method for configuring a neurostimulation device according to aspects of the present disclosure.

### Detailed description of illustrative examples

While specific feature combinations are described in the following with respect to exemplary aspects of the present disclosure, it is to be understood that not all features of the described aspects must be present for realizing the technical advantages provided by the devices, systems, methods and computer-readable media provided by the present disclosure. The disclosed examples may be modified by combining certain features of one example with one or more features of other examples if technically feasible and functionally compatible. Specifically, the skilled person will understand that features, steps, components and / or functional elements of one example can be combined with technically compatible features, steps, components and / or functional elements of any other example disclosed herein.

Moreover, the described components, modules, functions and / or functional elements of the devices and systems disclosed herein can be implemented in hardware, in software, or a combination thereof. For instance, the described components, modules, functions and / or functional elements of the devices and systems disclosed herein may be implemented via application specific hardware components such as application specific integrated circuits, ASICs, and / or field programmable gate arrays, FPGAs, and / or similar components and / or application specific software modules, applications, or subroutines being executed on multi-purpose data and signal processing circuitry such as CPUs, DSPs and / or systems on a chip (SOCs) or similar components or any combination thereof. For instance, the various components, modules, functions and / or functional elements, etc. described herein may be implemented on a multi-purpose data and signal processing device configured for executing application specific software modules and for communicating with various sensor devices and / or neurostimulation equipment or systems via conventional wireless communication interfaces such as a Near Field Communication (NFC), Transcutaneous optical telemetry, a WIFI and / or a Bluetooth interface. For example, aspects of the programmer devices described herein may be implemented on a smart phone or a tablet computer or a similar computing device. Alternatively, the various components, modules, functions and / or functional elements described herein may also be part of an integrated neurostimulation device, apparatus or system, comprising specialized electronic circuitry (e.g., neurostimulation signal generators, amplifiers etc.) for generating and applying the determined neurostimulation sequences to a neurostimulation interface of a person (e.g., a multi-contact electrode, a spinal cord stimulation electrode, a DBS electrode etc.).

The term "quiescence time", QT, as used herein is a time period in which a signal is non-active (e.g., interrupted, disabled etc.). The term "based on" as used herein, shall not be construed as a reference to a closed set of information, one or more conditions, one or more factors, or the like. In other words, the phrase "based on A" (where "A" may be information, a condition, a factor, or the like) shall be construed as "based at least on A" unless specifically recited differently. When reference is made herein to a "component", "unit", "device" or the like, this should not be understood as limiting to a particular "component", "unit", "device" or the like, but should encompass other implementation that could have similar and/or the same functions.

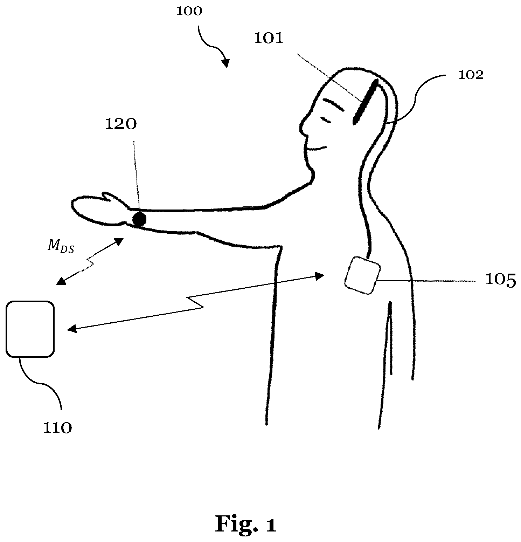

**Fig. 1** shows a schematic illustration of a system for configuring a neurostimulation device 105 according to aspects of the present disclosure. A person 100 is depicted, which has been implanted with one or more stimulation electrodes 101, which are in conductive connection with one or more stimulation leads 102. The stimulation electrode 101 may be a DBS electrode, a spinal cord stimulation electrode, a peripheral nerve electrode, a cortical stimulation electrode or the like having one or more stimulation channels. As an example, the stimulation electrode 101 may be already implanted into the brain of the person 100 in order to treat a neurological disorder of the person 100, such as Parkinson's disease, dystonia, and/or essential tremor or a similar disorder. The stimulation electrode 101 may also be used for communicating information to the brain, e.g., as part of a CBI device or system.

The person 100 is further equipped with a neurostimulation device 105, that may be an implantable and programmable pulse generator (IPG), which may be implanted under the skin of the person 100. Alternatively, the neurostimulation device 105 may be arranged somewhere else or in the vicinity of the body of the person 100. The neurostimulation device 105 may be in wireless communication (e.g., via a Bluetooth, WI-FI, NFC or a similar wireless interface technology) with a control device, that may be implemented by a dedicated signal and data processing device such as a smartphone or a similar electronic information processing device.

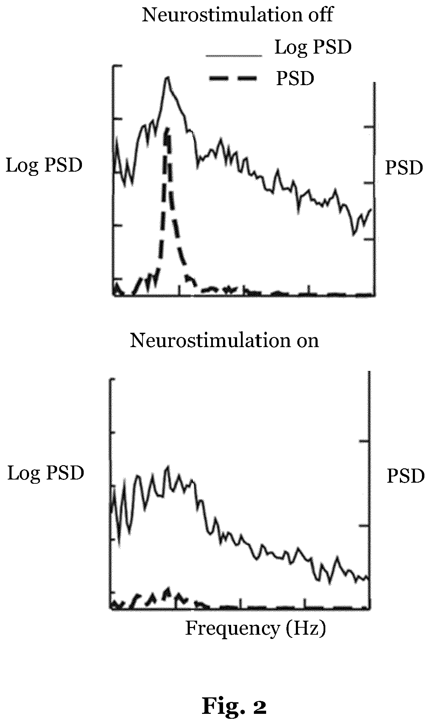

**Fig. 1** also depicts a programmer device 110 for configuring the neurostimulation device 105 which may be comprised by the control device or a separate device such as a personal computer, tablet computer or the like. The programmer device 110 may exchange data and control instructions with the neurostimulation device 105, for example via a wireless communication interface as described above. Furthermore, a sensor 120 (such as an accelerometer, a force, torque, and/or strain sensor or a similar sensor), is shown that can be used for determining a neurophysiologic effect strength *M_{DS}*, e.g., via measuring a physiologic parameter of the person 100 indicating a neurologic disorder strength (DS). The same or similar sensors can also be used to determine a side effect strength as discussed in more detail elsewhere herein. For instance, for determining the strength of typical symptoms of essential tremor, the sensor 120 may be an accelerometer (e.g., included in a smart watch) measuring tremor induced muscle contractions (e.g., indirectly via measuring movements caused by the tremor induced muscle contractions). For example, an output signal of the accelerometer may be used to determine or record a power spectral density (PSD) of tremor-induced muscle contractions (see Fig. 2). Similarly, electromyography signals may be obtained, e.g., via a smart watch with surface electrodes or similar sensor equipment.

In further aspects, sensing equipment for recording electro-physiological data such as EMG, spike patterns and/ or local field potential recordings from the central (CNS) and / or the peripheral nervous system (PNS) may be used to determine neurophysiologic effect strength, side effect strength, or both. Further, the programmer device 110 may be configured to cause the neurostimulation device 105 to generate a neurostimulation sequence adapted to cause a desired neurophysiologic effect, e.g., to treat the neurological disorder resulting in a reduction of the neurologic disorder strength, DS. For instance, the programmer device 110 may transmit a set of parameters like pulse frequency, pulse amplitude, pulse shape, pulse timing, duty cycle, etc. to the neurostimulation device that define a specific neurostimulation sequence to be generated and applied by the neurostimulation device 110 to the CNS and / or the PNS of the person 100 to cause the desired neurophysiologic effect, e.g., to suppress a symptom caused by a neurological disorder such as tremor-induced muscle contractions. At the same time, a physiologic parameter of the person 100 indicating the neurophysiologic effect strength and / or the side effect strength may be measured via the sensor 120 or further sensors (not shown).

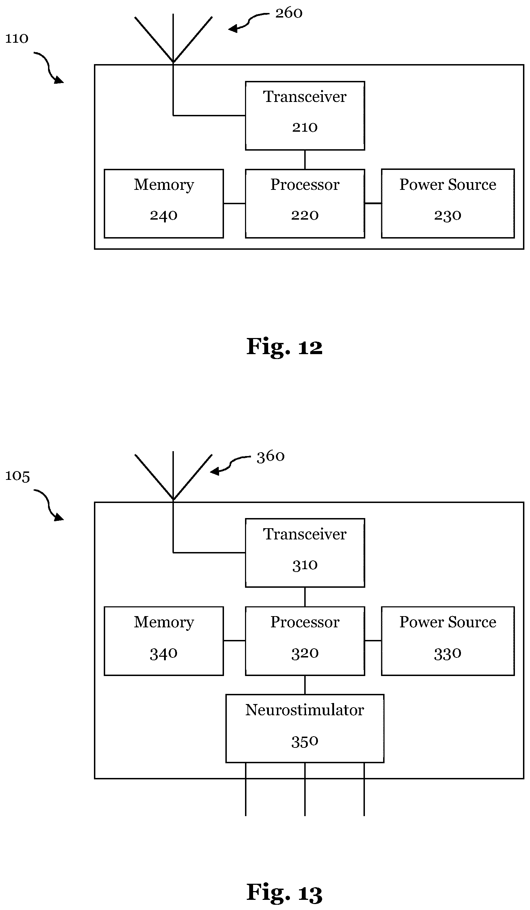

The programmer device 110 may be further configured to generate, based on the measured parameter *M_{DS}* indicating neurophysiologic effect strength, a modified neurostimulation sequence, e.g., via executing an iterative and closed-loop procedure as described below, which comprises an increased quiescence time QT of the neurostimulation sequence. The modified neurostimulation sequence may thus constitute an updated/improved neurostimulation sequence that induces less side effects while still causing the desired neurophysiologic effect, such as eliciting an artificial sensation / sensory percept to communicate conceptual information to the brain of the person with high fidelity. The programmer device 110 may be further configured to store the modified neurostimulation sequence in a non-transitory memory medium of the neurostimulation device 105. As described below with reference to Fig. 12, the modified neurostimulation sequence(s) can thereby be saved and conveniently be used for future applications in case the need arises.

**Fig 2** illustrates a power spectral density (PSD) derived from an exemplary output signal of an accelerometer measuring (e.g., indirectly as discussed above) tremor symptom strength such as tremor-induced muscle contractions. The PSD refers to a spectral energy distribution in frequency per unit of time. The total power within a given frequency range can thus be determined by integrating the PSD. In case the neurologic disorder is essential tremor, an accelerometer may be applied to measure the disorder strength. A higher PSD indicates that the disorder strength is increased (e.g., the symptoms become worse). For instance, the peak in the upper panel is present when the DBS used to treat tremor is turned off. For instance, the peak may correspond to a dominant frequency of tremor-induced muscle contractions caused by pathologically active motor regions of the CNS.

The lower panel illustrates the effect of an effective DBS therapy. The peak present in the upper panel is gone and the background signal is reduced. As can be understood from this example the integrated PSD of the output signal of an accelerometer or a similar sensor measuring for example tremor-induced muscle contractions or similar physiologic symptoms can thus be used to quantify / determine the strength of the neurophysiologic effect.

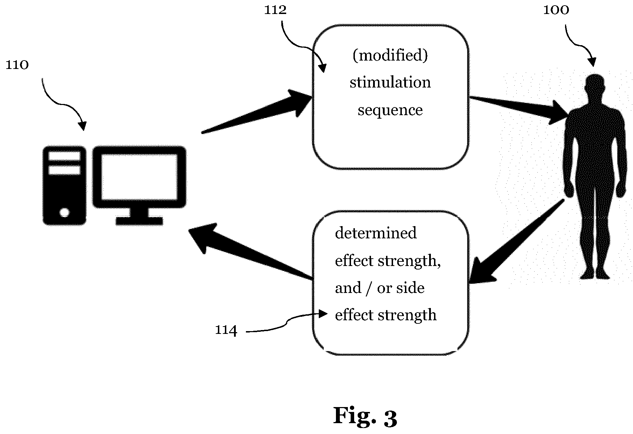

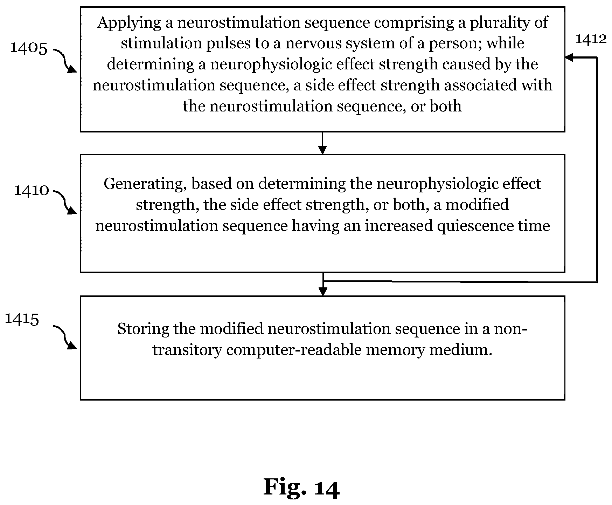

**Fig. 3** shows a schematic block diagram of a method and system for configuring a neurostimulation device according to aspects of the present disclosure. The programmer device 110 may operate in a closed-loop configuration with the sensor and the neurostimulation device of the person 100 (see Fig 1). A neurostimulation sequence is generated by the programmer device 110 and applied to the person 100 via a neurostimulation device. A parameter indicating neurophysiologic effect strength is measured by the sensor and sent to the programmer device 110 which is configured to generate or cause the neurostimulation device to generate, based on the measured parameter, a modified neurostimulation sequence which is applied to the CNS and / or the PNS of the person while measuring the parameter indicating neurophysiologic effect strength. Specifically, the system shown in Fig. 3 may be configured to carry out the steps of the methods discussed in section 3 above and with reference to Fig. 14 below.

The process illustrate in Fig. 3 may be repeated multiple times to arrive at a (optimized) modified neurostimulation sequence with increased quiescence time to reduce side effects caused by the neurostimulation sequence. As discussed below in detail with reference to Fig. 14, depending on a desired result, a system comprising programmer device, sensor and neurostimulation device may be configured to generate the modified neurostimulation sequence based on specific constraints and/or thresholds such as a preconfigured threshold for the measured parameter indicating neurophysiologic effect strength.

According to aspects of the present disclosure the programmer device may be operably connected to the sensor and the neurostimulation device and be adapted for causing the neurostimulation device and the sensor to generate, based on the determined neurophysiologic effect strength the determined side effect strength, or both, a modified neurostimulation sequence having an increased quiescence time, wherein generating the modified neurostimulation sequence comprises reducing a number of stimulation pulses of the neurostimulation sequence. As discussed in more detail below, generating the modified neurostimulation sequence may further comprise increasing one or more of a pulse frequency, a pulse amplitude or the pulse width of the neurostimulation sequence to increase a charge injected by the neurostimulation sequence into the nervous system as compared to the neurostimulation sequence with reduced number of stimulation pulses. The programmer device may then cause the neurostimulation device to store the modified neurostimulation sequence in a non-transitory computer-readable memory medium, e.g., of the programmer device or the neurostimulation device.

In some aspects, generating and storing the modified neurostimulation sequence may comprise: reducing the number of stimulation pulses of the neurostimulation sequence, and applying, using the neurostimulation device, the modified neurostimulation sequence to the nervous system of the person while determining the neurophysiologic effect strength, and determining whether the neurophysiologic effect strength remains above a preconfigured threshold for the neurophysiologic effect strength while the modified neurostimulation sequence is applied, and based on the determination whether the neurophysiologic effect strength remains above the preconfigured threshold, further modifying the neurostimulation sequence or storing the modified neurostimulation sequence in the non-transitory computer-readable memory medium. Alternatively, or additionally, as discussed below with reference to Fig. 14, similar steps may also be carried out based on a determined side effect strength that remains below a preconfigured threshold for the side effect strength. In other words, an initial neurostimulation sequence may be iteratively modified to optimize the quiescence time under the constraint that the neurophysiologic effect strength remains above a preconfigured threshold and / or under the constraint that the side effect strength remains below a preconfigured threshold.

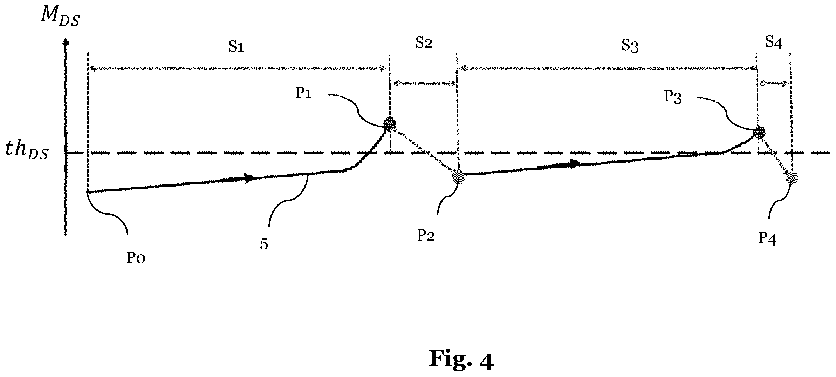

**Fig. 4** shows a schematic diagram illustrating how a modified neurostimulation sequence may iteratively be generated while measuring a physiologic parameter *M_{DS}* indicating strength of a desired neurophysiologic effect. The y-axis of the diagram shows the measured parameter *M_{DS}* indicating the neurophysiologic effect strength. For example, *M_{DS}* may be an integrated power spectral density of an output signal of an accelerometer measuring tremor-induced movements of a body part of the person as described with reference to Fig. 2 above. In other examples, *M_{DS}* may also quantify a subjective experience of the person characterizing the intensity, quality or presence of one or more artificial sensations elicited by the neurostimulation sequence. For example, *M_{DS}* may be a binary indication whether such an artificial sensation can be correctly perceived by the person or not.

A preconfigured threshold *th_{DS}* for the determined neurophysiologic effect strength is indicated in Fig. 4 as a dashed line. For example, the threshold *th_{DS}* may correspond to a physiology disorder strength, e.g., a tremor intensity, e.g., an intensity of tremor-induced movements or similar symptoms, which should not be exceeded, and which may serve as a constraint for determining the modified neurostimulation sequence that reduces side effects. The trajectory 5 may correspond to the time evolution of the measured physiologic parameter indicating neurophysiologic effect strength while the neurostimulation sequence is being modified, e.g., via an iterative adjustment procedure as discussed elsewhere herein (e.g., see Fig. 14). For example, the trajectory 5 may correspond to the time evolution of an integrated power spectral density of an output signal of an accelerometer measuring tremor intensity, e.g., tremor-induced movements.

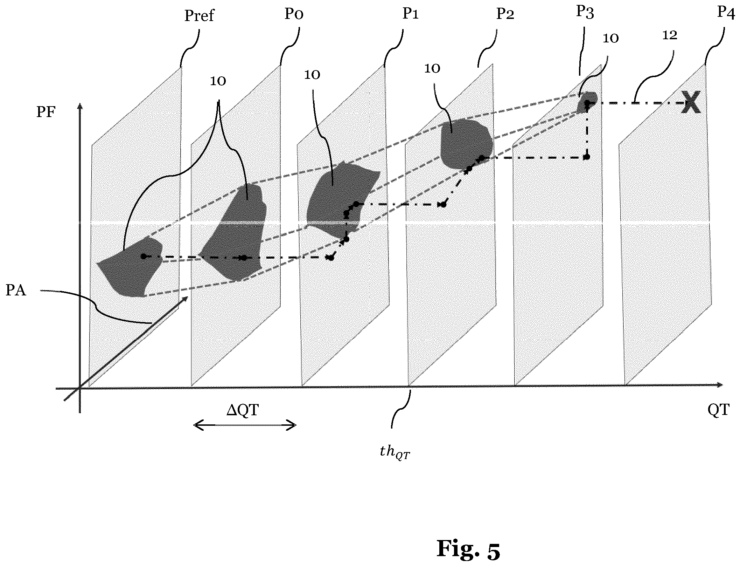

For instance, in a first time period S1 the quiescence time of the neurostimulation signal may be iteratively increased as discussed in detail with reference to Fig. 5, Fig. 7 and Fig. 8 below. Typically, as a result, the charge injected into the CNS of the person by the iteratively modified neurostimulation sequence is reduced and treatment effectiveness and / or efficacy e.g., symptom suppression effectiveness and / or efficacy) of the neurostimulation sequence iteratively decreases. Said decrease in treatment effectiveness and / or efficacy typically leads to an iteratively increasing value for the measured physiologic parameter from a starting or reference value P0 to a value P1 that lies above the preconfigured threshold *th_{DS}.* In this example, the measured physiologic parameter (e.g., an integrated PSD of an accelerometer) has an inverse relationship to the strength of the desired neurophysiologic effect, i.e., the measured physiologic parameter raises above the threshold, when the desired effect strength falls below a corresponding threshold. In other examples, e.g., when an intensity of an elicited artificial sensation is measured / characterized the relationship is not inverse. According to aspects of the present disclosure, the programmer device discussed above may determine, based on the measured physiologic parameter, that the threshold is exceeded and in response may cause the neurostimulation device, during a second time interval S2, to iteratively increase one or more of a pulse frequency, a pulse amplitude or a pulse width of the neurostimulation sequence such that the charge injected by the neurostimulation sequence into the nervous system is increased until the measured physiologic parameter falls below the preconfigured threshold *th_{DS}* for the measured physiologic parameter to a value P2.

This process may be repeated during time intervals S3 and S4 until the quiescence time of the neurostimulation sequence is above a preconfigured threshold for the quiescence time or until the quiescence time is optimized within safe operation parameters of the neurostimulation device and under the optimization constraint that the measured physiologic parameter remains below the preconfigured threshold *th_{DS}* for the measured physiologic parameter. As discussed above, the corresponding strength of the desired neurophysiologic effect may fulfill the inverse constraint, i.e., to remain above a preconfigured threshold for the neurophysiologic effect strength. Further, alternatively or additionally, as discussed above and with reference to Fig. 14 below, according to aspects of the present disclosure, a similar procedure may also be carried out based on determining the side effect strength and a respective threshold for the side effect strength.

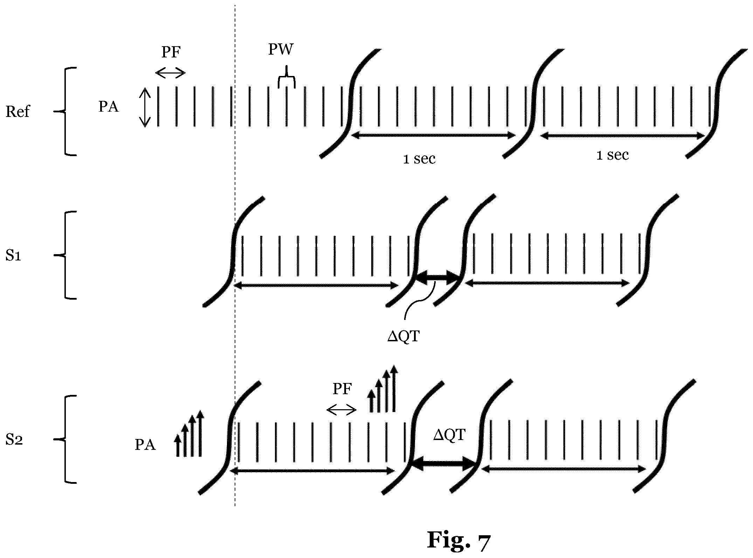

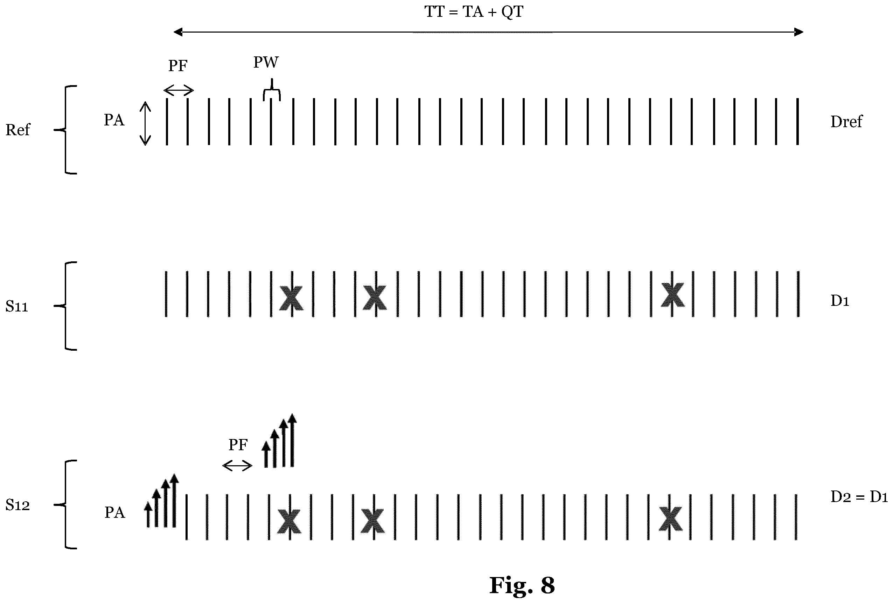

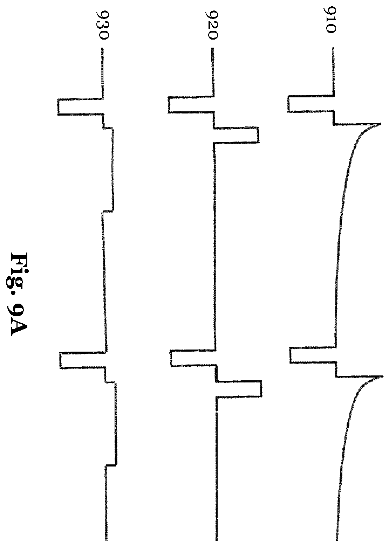

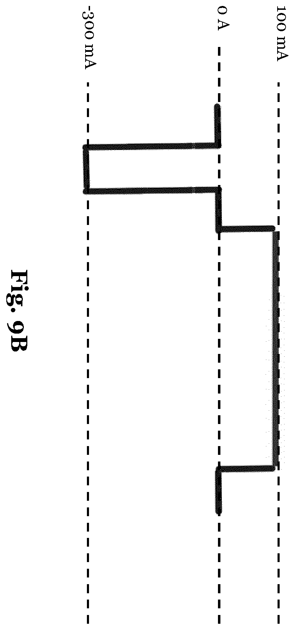

In some aspects, during time intervals S1, S3, etc. the number of stimulation pulses may iteratively be reduced by including one or more quiescence periods of increasing length into the neurostimulation sequence (see Fig. 7). Alternatively, or additionally, the number of stimulation pulses may be iteratively reduced by randomly or pseudo-randomly removing stimulation pulses from the neurostimulation sequence based on an iteratively increasing stimulation pulse removal probability (see Fig. 8). Alternatively, or additionally, the pulse shape of one or more stimulation pulses may be modified. As explained below with reference to Fig. 9A and Fig. 9B, an active asymmetric recharge pulse-shape may also be used for side-effect reduction.

**Fig. 5** shows a further schematic diagram illustrating how aspects of the present disclosure allow to configure a neurostimulation device with a modified stimulation sequence that causes less side effects. Here, the x-axis corresponds to a quiescence time QT of the neurostimulation sequence. In the illustrated example, the y-axis corresponds the pulse frequency (PF), and the z-axis to the pulse-amplitude (PA) of the neurostimulation sequence. In other examples, the y-axis or z-axis may correspond to the pulse width of the neurostimulation sequence or other signal parameters that affect the charge injected. In general, increasing the pulse frequency, the pulse amplitude and / or the pulse width results in an increase of charge injected by the neurostimulation sequence into the CNS and / or the PNS of the person, e.g., during time intervals where the neurostimulation sequence is not paused (see Fig. 7). Exemplarily, to illustrate the method and system described herein, the neurostimulation sequence may have a quiescence time QT according to the plane indicated by P0 of Fig. 5. At such a value for QT, the neurostimulation sequence may be able to treat a neurologic disorder of a person such that the neurophysiologic effect strength is above a preconfigured threshold.

**Fig. 5** shows six grey-shaded planes that correspond to six different values Pref, P0, P1, P2, P3, P4 of QT of the neurostimulation sequence modified, for instance, via reducing a number of stimulation pulses as explained elsewhere herein. For each value of QT, the dark-shaded regions 10 represents parameter combinations for which the neurophysiologic effect strength remains above a predefined threshold as discussed with reference to Fig. 4. Said parameter combinations can thus be used, for each given value of the quiescence time QT, to cause the desired neurophysiologic effect (e.g., treat the neurologic disorder) with sufficient effectiveness and / or efficacy. For instance, the dark-shaded regions 10 may correspond to combinations of pulse frequency and pulse amplitude that sufficiently suppress tremor-induced muscle contractions or that consistently evoke a desired artificial sensation perceivable by the person.

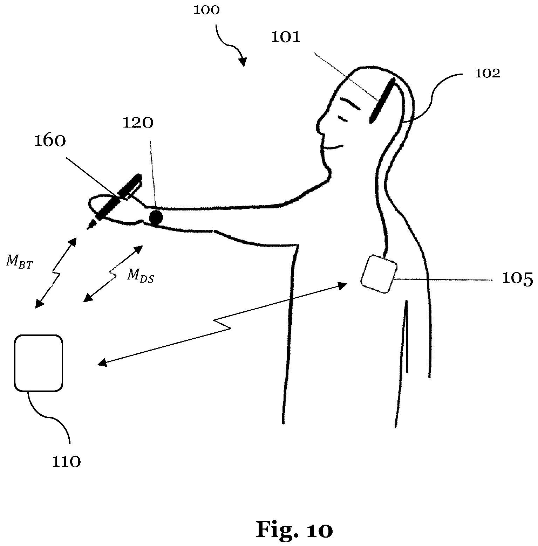

Outside the regions 10, the charge injected by the neurostimulation sequence may be too low for sufficient treatment of the neurologic disorder, such that the neurophysiologic effect strength is below the predefined threshold. It may also be possible that outside the regions 10, a side effect strength associated with the neurostimulation sequence may become too large or the person may experience pain or similar discomfort or a safety limit for the output of the stimulation device has been reached. As explained with reference to Fig. 10 below, aspects of the present disclosure also allow measuring / determining a side effect strength in addition to the neurophysiologic effect strength, e.g., via measuring a parameter indicating a degree of successful execution of a behavior task by the person affected by the side effects. Thus, determining the modified neurostimulation sequence as described herein may additionally or alternatively be based on a determined side effect strength. Increasing the quiescence time by ΔQT without increasing PF and / or PA may result in insufficient effect strength as indicated by the trajectory 12. Here, the neurophysiologic effect strength falls below the predefined threshold. Subsequently, the parameters PF and / or PA may be increased, thereby increasing the charge injected by the neurostimulation sequence such that effect strength is restored and the effect strength raises above the predefined threshold.

The steps described herein may be repeated in an iterative manner until the quiescence time QT of the neurostimulation sequence is above a predefined threshold *th_{QT}* for the quiescence time. It may also be feasible that the steps are repeated until the quiescence time QT is maximized meaning that if QT is increased further, it is not possible to restore effect strength as shown in plane P4. In some situations, it might also be beneficial to reduce the quiescence time QT between some iterations, e.g., to identify a different combination of pulse frequency, pulse amplitude and / or pulse width for which QT may be increased further while the effect strength and / or the side effect strength remain above or below their respective threshold. Thus, the present disclosure is not limited to stimulation sequence configuration systems and methods where QT always increases from one iteration to the next.

In this manner, aspects of the present disclosure allow to identify a plurality of different neurostimulation sequences that exhibit sufficient effect strength and / or low enough side effects at various values for the quiescence time QT in a patient-specific manner. Some or all identified neurostimulation sequences can then be stored in a memory of the neurostimulation device for further use (e.g., for treatment of a neurologic disorder or CBI applications, etc.). For instance, as discussed below, stored sequences may be applied based on an input signal. Such an input signal may comprise a randomly generated selection instruction, or a selection instruction derived from a preconfigured therapy schedule or a user input etc.

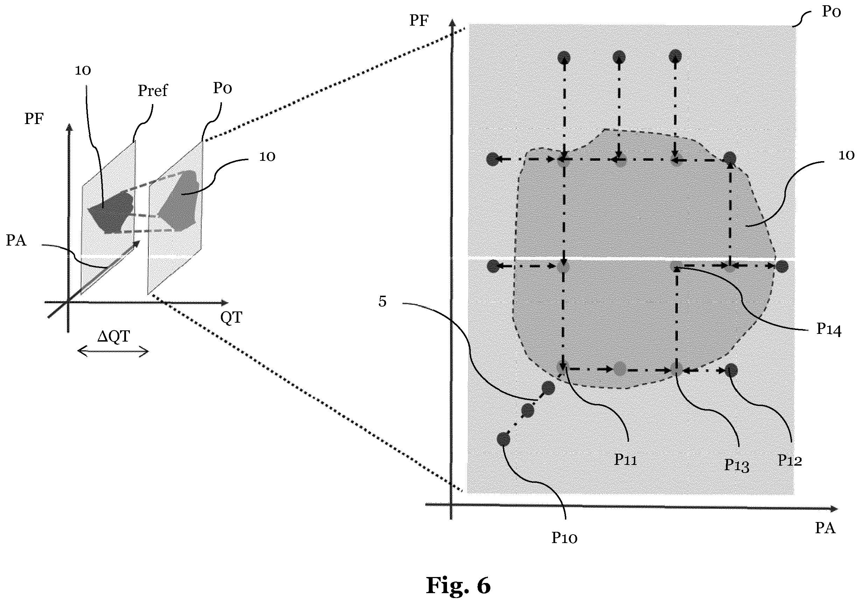

**Fig. 6** shows an excerpt of Fig. 5 in greater detail. The plane P0 of constant quiescence time is shown in greater detail on the right-hand side of the illustration. It may be possible that after increasing the QT from Pref to P0, the location of the trajectory 12 may be at value P10 outside the dark-shaded region 10. Since this may be undesired, the pulse amplitude PA (x-axis) and/or the pulse frequency PF (y-axis) may be iteratively increased, such that the trajectory 12 is located at P11 within the region 10. To identify the parameter space 10 in greater detail and/or with a higher resolution, variations of PA and/or PF may be performed to characterize the boundary of the region 10. As an example, the pulse amplitude PA may be increased by a predefined step-size, such as 0.5 mA or 1.0 mA or the like. As shown, the pulse amplitude PA is increased in three steps to P12, i.e., outside region 10. Upon this, the pulse amplitude is reduced, such that the trajectory is at P13. Then, the pulse frequency may be increased, e.g., by a predefined step-size, such as 1 Hz, 5 Hz, 10 Hz or the like. As shown, the pulse frequency may be increased to P14 and so on. These steps may be repeated with smaller or larger step sizes such that the region 10 is characterized with a desired accuracy. The described aspects offer improved flexibility, as the charge injected by the neurostimulation sequence may be a function of pulse amplitude, pulse frequency (and also pulse width). Thereby, an optimum working area / parameter space for the neurostimulation sequence may be identified with increased QT as compared to Pref, while ensuring that the neurophysiologic effect remains above the preconfigured threshold. Aspects of the present disclosure thus allow to determine stimulation parameter combinations that lie well within the region 10 and thus allow for stable operation of neurostimulation device under changing external conditions or patient conditions (e.g., increased medication, etc.).

**Fig. 7** illustrates how the quiescence time of an initial neurostimulation sequence Ref can iteratively be increased by including one or more quiescence periods of increasing length ΔQT into the neurostimulation sequence while also increasing amplitude, frequency and/or pulse width of some or all pulses of the neurostimulation sequence. For example, the initial neurostimulation sequence may be subdivided into a plurality of segments of a certain duration, such as 100 ms, 1 s or 10s. Each segment may comprise a plurality of neurostimulation pulses having a pulse amplitude PA, a pulse frequency PF and a pulse width PW.

In a first iteration, a first modified neurostimulation sequence S1 may be generated, while determining a neurophysiologic effect strength (e.g., via measuring a physiologic parameter of the person indicating a neurologic disorder strength, etc.) by including one or more quiescence periods of length ΔQT (e.g., a certain percentage of segment duration, e.g., 1% of a 1 second segment equals 10ms, etc.) into the initial neurostimulation sequence Ref. For example, a quiescence period of length ΔQT may be included in between subsequent pairs of segments, or may be (pseudo)-randomly inserted into the sequence. In this manner, the quiescence time of the neurostimulation sequence may be increased while the neurophysiologic effect strength remains above the preconfigured threshold and / or the side effect strength remains below a preconfigured threshold, or both as also discussed above with reference to Fig. 4 to 6.

In a next iteration the quiescence time of the neurostimulation sequence S1 may be further increased to generate a second modified neurostimulation sequence S2 for which the neurophysiologic effect strength may fall below the preconfigured threshold. To restore the desired effect the PA and / or the PF and / or the PW of the stimulation pulses within the segments may be increased as discussed in detail with reference to Figs. 5, 6 and 7 above. These steps may be repeated until the quiescence time of the neurostimulation sequence is above a preconfigured threshold for the quiescence time or until the quiescence time is maximized (under the constraint that the neurophysiologic effect strength ultimately remains above the preconfigured threshold and / or the side effect strength remains below a preconfigured threshold). The resulting (set of) modified neurostimulation sequence(s) may be stored for further use in a memory of the neurostimulation device or elsewhere.

**Fig. 8** illustrates how the quiescence time QT of an initial neurostimulation sequence Ref can iteratively be increased by reducing the number of stimulation pulses by randomly or pseudo-randomly removing stimulation pulses from the neurostimulation sequence based on an iteratively increasing stimulation pulse removal probability in a closed-loop manner, based on a determining the neurophysiologic effect strength, the side effect strength or both while also increasing amplitude, frequency and/ or pulse width of some or all pulses of the neurostimulation sequence.

In a first iteration a first modified neurostimulation sequence S11 may be generated, while determining the neurophysiologic effect strength, the side effect strength or both by randomly or pseudo-randomly removing stimulation pulses based on a stimulation pulse removal probability. For instance, the probability for removal of stimulation pulses may be 2.5%. In this manner, the quiescence time of the neurostimulation sequence may be increased while the neurophysiologic effect strength remains above a preconfigured threshold, the side effect strength remains below a preconfigured threshold, or both as also discussed above with reference to Figs 4 to 6.

In a next iteration the quiescence time of the neurostimulation sequence may be further increased using a larger probability for removal of stimulation pulses e.g., 5% to generate a second modified neurostimulation sequence S12 for which the neurophysiologic effect strength may fall below the preconfigured threshold. To restore the desired effect, the PA and / or the PF and / or the PW of the non-removed stimulation pulses may be increased as discussed in detail elsewhere herein. These steps may be repeated until the quiescence time of the neurostimulation sequence is above a preconfigured threshold for the quiescence time or until the quiescence time is maximized (under the constraint that the neurophysiologic effect strength ultimately remains above the preconfigured threshold and / or the side effect strength remains below a preconfigured threshold) and the resulting (set of) modified neurostimulation sequence(s) may be stored for further use in a memory of the neurostimulation device or elsewhere.

**Fig. 9A** shows a schematic illustration of a neurostimulation sequence comprising multi-phasic and asymmetric pulses. For illustration, the top trace 910 in Fig. 9A shows a conventional mono-phasic stimulation sequence with passive recharge behavior. The middle trace 920 shows a bi-phasic sequence wherein each pulse is symmetric and comprises a stimulation phase (negative polarity) and an active charge balancing phase (positive polarity). The bottom trance 930 illustrates an example of a modified stimulation sequence according to aspects of the present disclosure wherein the modified stimulation sequence comprises multi-phasic stimulation pulses, wherein each multi-phasic stimulation pulse comprises a stimulation phase (negative polarity) and one or more active charge balancing phase (positive polarity), wherein an amplitude of the active charge balancing phase is reduced and a duration of the active charge balancing phase is increased with respect to the symmetric pulse of the sequence 920.

Using one or multiple active recharge period(s) / phases which are lower in intensity with respect to the stimulation phase but their total pulse width is greater than the initial phase allows to keep the pulse charge balanced while further reducing side effects. For example, the active recharge period can reduce the total length of pulse duration when compared to the conventional passive discharge method as it does not depend on the passive discharge time constant. This can increase possibility of fitting pulses with higher stimulation frequency in a burst. In addition, limiting the anodic (or cathodic) pulse amplitude can contribute to reduction of the inhibitory effects of stimulation or in other words, it can reduce the side-effects of therapy normally caused by blocking the natural neuronal activity in the CNS and / or the PNS, e.g., depending on parameters such as the implant site, the orientation and distance of nerve fibers with respect to the lead. Fig. 9B shows a zoom-in on one of the stimulation pulses of the sequence 930 of Fig. 9A with typical current values.

**Fig. 10** shows a schematic illustration of a method and system for configuring a neurostimulation device 105 according to an aspect of the present disclosure describing further details of a closed-loop configuration. The following description mainly focuses on differences to the description of the example of Fig. 1.

As discussed above, a side effect strength (as an alternative to the strength of the desired neurophysiologic effect or in addition) associated with the neurostimulation sequence may be determined and used for generating the modified stimulation sequence as described above. For example, determining the side effect strength may comprise measuring a parameter *M_{BT}* indicating a degree of successful execution of a behavior task 160 by the person 100. In the shown example, the behavior task comprises a drawing task, which may be negatively affected by side effects caused by certain neurostimulation treatment paradigms, e.g., by neurostimulation sequences configured to reduce tremor-induced muscle contractions. For example, the person 100 may be instructed to draw a circle or write his name using a sensor equipped pen 160 while the neurostimulation device 105 is configured and operated as described elsewhere herein. The output signal of the sensor equipped pen 160 or of an imaging system or similar sensor equipment may be used to derive the parameter *M_{BT}* indicating a degree of successful execution of the drawing task. Since the execution of the drawing task may be negatively affected by typical side effects that may be caused by the neurostimulation sequences applied to the nervous system, the parameter *M_{BT}* can be used to enhance the closed-loop configuration method for the neurostimulation device 105. In other examples, the parameter *M_{BT}* may also provide an indication of the neurophysiologic effect strength and thus be used in a similar manner as discussed above. For example, analysis of a drawing task or a similar task may allow to determine both, a neurophysiologic effect strength and a side effect strength.

As an example, treating a neurologic disorder of the person 100 by way of the neurostimulation device 105 may cause side effects, leading to a reduction of the measured parameter *M_{BT}.* For instance, the person 100 may be under-performing in a drawing task. For example, this additional information may be used to further refine the modified stimulation sequence, e.g., by informing the programmer device 110 of negative or positive effects of stimulation parameters that are iteratively modified as discussed above.

In some aspects, the programmer device 110 may be configured to automatically check the measured parameters *M_{DS}* and *M_{BT}* at specified time intervals, which could be defined by medical personnel and/or the person 100. Accordingly, the programmer device 110 may be configured to generate a modified neurostimulation sequence at specified time intervals and / or if the measured parameters *M_{DS}* and *M_{BT}* indicate that a modification may be beneficial for the person 100.

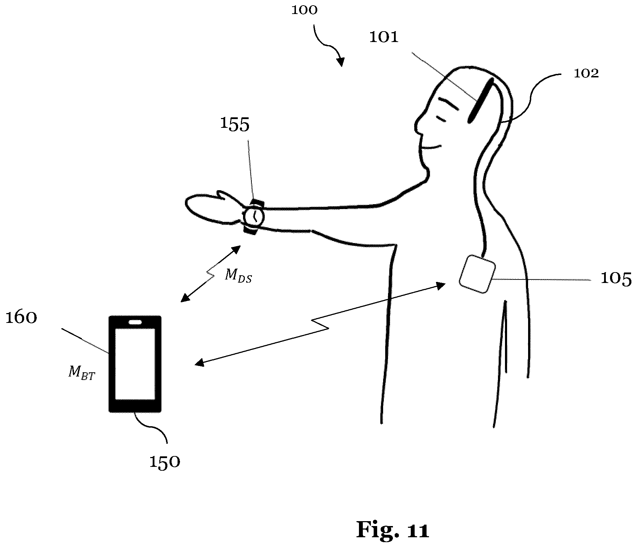

As shown in **Fig. 11** the task 160 can also be displayed or executed on a smart phone or tablet computer 150, which may also serve as the programming device disclosed herein (see Fig. 12), e.g., communicating with a stimulation device 105. Further, the smart phone or tablet computer 150 may also receive measurements M_{DS} such as accelerometer signals from a smart watch 155 characterizing a strength of a desired neurophysiologic effect as discussed in detail above.

**Fig. 12** shows a functional block circuit diagram illustrating a programmer device 110 according to aspects of the present disclosure, such as a smart phone, or similar computing device. The programmer device 110 may comprises a communication antenna 260 operably connected to a transceiver 210, configured for wireless communication (e.g., via NFC, Bluetooth or a similar wireless or optical communication technology). The transceiver 210 may be configured, for example, to receive one or more sensor signals from one or more sensors as described herein, that may be indicative of a neurophysiologic effect strength, a side effect strength, or both (e.g., an acceleration signals obtained from an accelerometer, a distances signal obtained from a motion tracking sensor device, etc.).

The transceiver 210 may be further configured to receive a signal indicative of a parameter indicating a degree of successful execution of a behavior task as discussed herein. The transceiver 210 may be operably connected to a processor 220, which may be configured to generate or to cause a neurostimulation device to generate an initial and one or more iteratively modified neurostimulation sequences, based on the received sensor signals and received parameters. As described in detail above, the generated neurostimulation sequences may be characterized by, a pulse frequency, a pulse amplitude, a pulse width, a quiescence time and/or the like.

The programmer device 110 may further comprise a memory 240, such as a non-transitory computer-readable memory. In some aspects, the neurostimulation sequences may be obtained from or stored in the memory 240. The processor 220 may additionally receive a neurostimulation sequence from the memory 240 and/or from the transceiver 210, wherein the received neurostimulation sequence may be used for generating modified neurostimulation sequences.

The memory 240 may further store instructions for causing the neurostimulation device and the sensor to carry out the steps of the methods disclosed herein, such as the steps of the method discussed with reference to Fig. 14 below.

The transceiver 210 may be further configured to output, e.g., send neurostimulation sequences or instructions for generating neurostimulation sequences via the communication antenna 260 to a neurostimulation device 105 as described below. The programmer device 110 may also comprise a power source 230 such as a rechargeable battery which may for instance be wirelessly charged via a wireless charging interface.

**Fig. 13** shows a functional block circuit diagram illustrating a neurostimulation device 105 according to aspects of the present disclosure. The neurostimulation device 105 may comprise a communication antenna 360 operably connected to a transceiver 310, configured for wireless communication (e.g., via NFC, Bluetooth or a similar wireless or optical communication technology).

The transceiver 310 may be configured to receive neurostimulation sequences or instructions for generating neurostimulation sequences sent from the programmer device 110 as described herein and/or from a remote-control device. The transceiver 310 may be operably connected to a processor 320, which may be configured to process or generate the neurostimulation sequences, e.g., based on instructions received via the transceiver and / or stored and obtained in a memory 340. The memory 340, such as a non-transitory computer-readable memory may also be configured to store one or more neurostimulation sequences. The memory 340 may be operably connected to the processor 320. The processor 320 may also receive neurostimulation sequences from the memory 340, e.g., for use in treating a neurologic disorder or for communicating information to the brain of the person, e.g., via eliciting artificial sensations carrying conceptual information as described in WO2020/174051 A1.

The neurostimulation device 105 may further comprises a neurostimulator 350, that receives the generated neurostimulation sequence from the processor 320. The neurostimulator 350 may also be connected to one or more output signal leads that may be in conductive communication with electrodes for applying the neurostimulation sequence to the CNS and / or the PNS of a person (e.g., a DBS electrode or a spinal cord stimulation electrode, a peripheral nerve stimulator etc.). The neurostimulation device 105 may also comprise a rechargeable power source 330, which may for instance be wirelessly charged via a wireless charging interface.

According to some aspects of the present disclosure, the processor 320 may thus be operably connected to neurostimulation circuitry adapted to generate and apply a plurality of neurostimulation sequences to a nervous system of a person. A non-transitory computer readable memory such as memory 340 may store a first and a second neurostimulation sequence, both comprising a respective plurality of stimulation pulses and adapted to cause a neurophysiologic effect, wherein the one or more second neurostimulation sequences may comprise a larger quiescence time than the first neurostimulation sequence by having less stimulation pulses, and wherein the second neurostimulation sequence(s) may comprises one or more of: an increased pulse frequency, an increased pulse amplitude or an increased pulse width, such that a charge injected by the second neurostimulation sequence is increased such as to compensate, at least partially, for the reduction in charge injected caused by having less stimulation pulses than the first sequence.

Further, the non-transitory computer readable memory 340 may store a plurality of second neurostimulation sequences each having a different quiescence time and a different combination of the increased pulse frequency, the increased pulse amplitude or the increased pulse width, and the processor 320 may be configured to select and apply one of the plurality of second neurostimulation sequences based on an input signal. For example, the input signal may comprise a user input. Further, the input signal may comprise a randomly generated selection instruction, or a selection instruction derived from a preconfigured therapy schedule. Further, the input signal may comprise a sensor signal indicative of one or more of: a neurophysiologic effect strength of the person, a side effect strength associated with a neurostimulation sequence currently being applied by the neurostimulation device, a somatic or psychologic state of the person (e.g., a sleep state), or an activity being carried out by the person (e.g., walking).

Further, the one or more second neurostimulation sequences may comprise multi-phasic stimulation pulses as described with reference to Fig. 9A and 9B above wherein each multi-phasic stimulation pulse comprises a stimulation phase and one or more active charge balancing phase, wherein the one or more active charge balancing phase of some or all of the multi-phasic stimulation pulses may comprise a lower amplitude than the stimulation phase and a longer duration than the stimulation phase. The one or more second neurostimulation sequences may be generated by the methods and systems as discussed herein, e.g., by using the neurostimulation device as part of a system as discussed above and / or via the method described with reference to Fig 14 below.

**Fig. 14** shows a process diagram illustrating an examplary method according to aspects of the present disclosure. For example, the method may be carried out by the exemplary system as described above. Step 1405 comprises applying, using a neurostimulation device, an neurostimulation sequence comprising a plurality of stimulation pulses to a nervous system of a person. For example, the neurostimulations sequence may be obtained from a non-transitory memory medium (such as the memory shown in Fig. 12 above) and be configured to cause a neurophysiologic effect such as treatment of a neurophysiologic disorder. While the neurostimulation sequence is being applied a neurophysiologic effect strength caused by the neurostimulation sequence, a side effect strength associated with the neurostimulation sequence, or both may be determined. For example, determining the neurophysiologic effect strength may comprise measuring, via a sensor device, a physiologic parameter of the person indicating the neurophysiologic effect strength. For example, the neurophysiologic effect may amount to a reduction of symptoms of a neurologic disorder.

Additionally, or alternatively, determining the neurophysiologic effect strength may comprise receiving a feedback signal from the person indicating the neurophysiologic effect strength. Further, the neurophysiologic effect may comprise one or more artificial sensations elicited by the neurostimulation sequence.

Additionally, or alternatively determining the neurophysiologic effect strength may comprises one or more of: measuring a parameter indicating a degree of successful execution of a behavior task by the person supported by the neurophysiologic effect, measuring neuronal activity of the person associated with the neurophysiologic effect, measuring a parameter indicating a psychological state of the person affected by the neurophysiologic effect, or determining a performance metric of a computer brain interface of the person using the neurophysiologic effect.

Additionally or alternatively, determining the side effect strength associated with the neurostimulation sequence may comprise one or more of: measuring a parameter indicating a degree of successful execution of a behavior task by the person affected by the side effect, measuring neuronal activity of the person associated with the side effect, measuring a parameter indicating a psychological state of the person affected by the side effect, or determining a performance metric of a computer brain interface of the person affected by the side effect.

Step 1410 comprises generating, based on determining the neurophysiologic effect strength, the side effect strength, or both, a modified neurostimulation sequence having an increased quiescence time. The step of generating the modified neurostimulation sequence may comprise reducing a number of stimulation pulses of the neurostimulation sequence by including one or more quiescence periods into the neurostimulation sequence, or by removing stimulation pulses from the neurostimulation sequence, or by doing both. The step of generating the modified neurostimulation sequence (step 1410) may further comprise increasing one or more of a pulse frequency, a pulse amplitude or a pulse width of the neurostimulation sequence to increase a charge injected into the nervous system of the person, e.g., to compensate, at least partially, the reduction in charge injected caused by increasing the quiescence time (see description Fig. 5 for further details).

Further, generating the modified neurostimulation sequence may comprise maximizing the quiescence time of the neurostimulation sequence under the constraint that the determined neurophysiologic effect strength remains above a preconfigured threshold for the neurophysiologic effect strength, or under the constraint that the determined side effect strength remains below a preconfigured threshold for the side effect strength, or under both constraints. As explained elsewhere herein (e.g., see Fig. 5) and indicated by arrow 1412 in Fig. 14 the steps of increasing the quiescence time and increasing the charge injected may be iterated until the quiescence time is optimized, wherein in each iteration step the modified sequence generated in the preceding step is applied to the nervous system of the person while the neurophysiologic effect strength and / or the side effect strength are determined. This optimization procedure may also be carried out in a (quasi)-continuous manner and the illustrated steps may be, at least partially, executed simultaneously.

For example, generating the modified neurostimulation sequence may comprise, reducing the number of stimulation pulses until the determined neurophysiologic effect strength falls below the preconfigured threshold for the neurophysiologic effect strength, or until the determined side effect strength raises above the preconfigured threshold for the side effect strength, or both; and increasing the charge injected by the neurostimulation sequence into the nervous system until the determined neurophysiologic effect strength raises above the preconfigured threshold for the neurophysiologic effect strength, or until the determined side effect strength falls below the preconfigured threshold for the side effect strength or both. For example, the number of stimulation pulses may iteratively be reduced by including one or more quiescence periods of increasing length into the neurostimulation sequence. For instance, the number of stimulation pulses may iteratively be reduced by randomly or pseudo-randomly removing stimulation pulses from the neurostimulation sequence based on an iteratively increasing stimulation pulse removal probability. In some examples the one or more quiescence periods may also be distributed randomly or pseudo-randomly (with a minimal distance between them) within the iteratively modified neurostimulation sequence in order to address certain habituation phenomena that may be detrimental to achieving the desired neurophysiologic effect.

In some aspects, generating the modified neurostimulation sequence may comprise repeating the reducing and increasing steps discussed above until the quiescence time of the neurostimulation sequence is above a preconfigured threshold for the quiescence time or until the quiescence time is maximized.

Further, according to some aspects, the parameter indicating the neurophysiologic effect strength may be measured via one or more of: an accelerometer attached to a body part of the person, an imaging system, or neural activity sensor, or a behavioral task. In some aspects, the parameter indicating the neurophysiologic effect strength may based on a power spectral density of an output signal of one or more of the accelerometer, the imaging system, or the neural activity sensor.

In other aspects, generating the modified neurostimulation sequence may comprise modifying a pulse shape of one or more stimulation pulses of the neurostimulation sequence. Alternatively, or additionally increasing one or more of the pulse frequency, the pulse amplitude or the pulse width of the neurostimulation sequence may be carried out such that the charge injected by the modified neurostimulation sequence into the nervous system is maintained or increased as compared to the non-modified sequence (e.g. an initial sequence or a sequence modified in a previous iteration).

In some aspects, the modified stimulation sequence may comprise multi-phasic stimulation pulses, wherein each multi-phasic stimulation pulse may comprise a stimulation phase and one or more active charge balancing phase, and wherein generating the modified stimulation sequence may further comprise modifying the shape of the one or more active charge balancing phase of some or all of the multi-phasic stimulation pulses. In some examples, modifying the shape of the active charge balancing phase of some or all of the multi-phasic stimulation pulses may comprise reducing an amplitude of the active charge balancing phase and increasing a duration of the active charge balancing phase.

At step 1415 the modified (e.g., iteratively as discussed above) neurostimulation sequence may be stored in a non-transitory computer-readable memory medium, e.g., of the programming device and / or the neurostimulation device described above. For example, after each iteration the (signal parameter of the) modified neurostimulation sequence may be stored in memory while the method may start again (1412) to further optimize the sequence. The method described above may also comprise applying the modified stimulation sequence to the nervous system of the person to cause a desired neurophysiologic effect with less side effects.

It is noted that the above examples may be combined with further aspects as described herein and details of the examples may also be omitted, as will be understood by the skilled person. The scope of protection is determined by the appended claims and is not limited to the examples discussed above.

## Claims

1. Computer program for carrying out the following steps when carried out by a processor (220, 320) of a neurostimulation system:
applying, using a neurostimulation device (105, 110), a neurostimulation sequence
comprising a plurality of stimulation pulses to a nervous system of a person (100);
while
determining a neurophysiologic effect strength caused by the neurostimulation sequence, a side effect strength associated with the neurostimulation sequence, or both; and
generating, based on determining the neurophysiologic effect strength, the side effect strength, or both, a modified neurostimulation sequence having an increased quiescence time; wherein generating the modified neurostimulation sequence comprises:
reducing a number of stimulation pulses of the neurostimulation sequence by including one or more quiescence periods into the neurostimulation sequence, or by removing stimulation pulses from the neurostimulation sequence, or by doing both; and
increasing one or more of a pulse frequency, a pulse amplitude or a pulse width of the neurostimulation sequence to increase a charge injected into the nervous system;
storing the modified neurostimulation sequence in a non-transitory computer-readable memory medium; and, optionally,
applying the modified stimulation sequence to the nervous system of the person (100) to cause the neurophysiologic effect.

2. Computer program of claim 1, wherein determining the neurophysiologic effect strength comprises measuring, via a sensor device, a physiologic parameter of the person (100) indicating the neurophysiologic effect strength; and / or wherein the neurophysiologic effect comprises a reduction of a neurologic disorder; and / or
wherein determining the neurophysiologic effect strength comprises receiving a feedback signal from the person (100) indicating the neurophysiologic effect strength; and / or wherein the neurophysiologic effect comprises one or more artificial sensations elicited by the neurostimulation sequence.

3. Computer program of any of claims 1 to 2, wherein determining the neurophysiologic effect strength comprises one or more of:
measuring a parameter indicating a degree of successful execution of a behavior task (160) by the person (100) supported by the neurophysiologic effect, measuring neuronal activity of the person (100) associated with the neurophysiologic effect, measuring a parameter indicating a psychological state of the person (100) affected by the neurophysiologic effect, or determining a performance metric of a computer brain interface of the person (100) using the neurophysiologic effect.

4. Computer program of any of claims 1 to 3, wherein determining the side effect strength associated with the neurostimulation sequence comprises one or more of:
measuring a parameter indicating a degree of successful execution of a behavior task (160) by the person (100) affected by the side effect, measuring neuronal activity of the person (100) associated with the side effect, measuring a parameter indicating a psychological state of the person (100) affected by the side effect, or determining a performance metric of a computer brain interface of the person (100) affected by the side effect.

5. Computer program of any of claims 1 to 4, wherein generating the modified neurostimulation sequence comprises:
maximizing the quiescence time of the neurostimulation sequence under the constraint that the determined neurophysiologic effect strength remains above a preconfigured threshold for the neurophysiologic effect strength; or under the constraint that the determined side effect strength remains below a preconfigured threshold for the side effect strength, or both.

6. Computer program of claim 5, wherein generating the modified neurostimulation sequence comprises:
reducing the number of stimulation pulses until the determined neurophysiologic effect
strength falls below the preconfigured threshold for the neurophysiologic effect strength, or until the determined side effect strength raises above the preconfigured threshold for the side effect strength, or both; and
increasing the charge injected by the neurostimulation sequence into the nervous system until the determined neurophysiologic effect strength raises above the preconfigured threshold for the neurophysiologic effect strength, or until the determined side effect strength falls below the preconfigured threshold for the side effect strength or both.

7. Computer program of claim 6, wherein the number of stimulation pulses is iteratively reduced by including one or more quiescence periods of increasing length into the neurostimulation sequence; and / or
wherein the number of stimulation pulses is iteratively reduced by randomly or pseudo-randomly removing stimulation pulses from the neurostimulation sequence based on an iteratively increasing stimulation pulse removal probability; and / or .
wherein generating the modified neurostimulation sequence comprises repeating the reducing and increasing steps of claim 6 until the quiescence time of the neurostimulation sequence is above a preconfigured threshold for the quiescence time or until the quiescence time is maximized.

8. Computer program of claims 2 to 7, wherein the parameter indicating the neurophysiologic effect strength is measured via one or more of: an accelerometer attached to a body part of the person (100), an imaging system, or neural activity sensor, or a behavioral task; and, preferably, wherein the parameter indicating the neurophysiologic effect strength is based on a power spectral density of an output signal of one or more of the accelerometer, the imaging system, or
the neural activity sensor.

9. Computer program of any of the preceding claims 1 to 8, wherein generating the modified neurostimulation sequence comprises modifying a pulse shape of one or more stimulation pulses of the neurostimulation sequence; and / or
wherein increasing one or more of the pulse frequency, the pulse amplitude or the pulse width of the neurostimulation sequence is done such that the charge injected by the modified neurostimulation sequence into the nervous system is maintained or increased.

10. Computer program of claim 9, wherein the modified stimulation sequence comprises multi-phasic stimulation pulses, wherein each multi-phasic stimulation pulse comprises a stimulation phase and one or more active charge balancing phase, and wherein generating the modified stimulation sequence further comprises:
modifying the shape of the one or more active charge balancing phase of some or all of the multi-phasic stimulation pulses; and, preferably, wherein modifying the shape of the active charge balancing phase of some or all of the multi-phasic stimulation pulses comprises:
reducing an amplitude of the active charge balancing phase and increasing a duration of the active charge balancing phase.

11. System comprising:
a neurostimulation device (105, 110) adapted to apply a plurality of neurostimulation sequences, each comprising a plurality of stimulation pulses to a nervous system of a person (100); and
a programming device, operably connected to the neurostimulation device (105, 110);
a memory (240, 340) storing the computer program of any of the claims 1 to 10, wherein the programming device and the neurostimulation device (105, 110) are adapted to carry
out the steps of the stored computer program.

12. Neurostimulation device (105, 110) comprising:
a processor (220, 320) operably connected to neurostimulation circuitry adapted to
generate and apply a plurality of neurostimulation sequences to a nervous system of a person (100); and
a memory (240, 340), operably connected to the processor (220, 320), storing a first and one or more
second neurostimulation sequences, each neurostimulation sequence comprising a respective plurality of stimulation pulses and being adapted to cause a respective neurophysiologic effect,
wherein the one or more second neurostimulation sequences comprise a larger quiescence time than the first neurostimulation sequence by having less stimulation pulses; and
wherein each of the one or more second neurostimulation sequences comprises one or more of an
increased pulse frequency relative to the first neurostimulation sequence, an increased pulse amplitude relative to the first neurostimulation sequence or an increased pulse width relative to the first neurostimulation sequence.

13. Neurostimulation device (105, 110) of claim 12, wherein the memory (240, 340) stores a plurality
of second neurostimulation sequences each having a different quiescence time and a different increased pulse frequency, increased pulse amplitude and / or increased pulse width; and wherein the processor (220, 320) is adapted to select and apply one of the plurality of
second neurostimulation sequences based on an input signal; and wherein, optionally, the input signal comprises one or more of a user input, a randomly generated selection instruction, or a selection instruction derived from a preconfigured therapy schedule; and, wherein, optionally, the input signal comprises a sensor signal indicative of one or more of: neurophysiologic effect strength of the person (100), a side effect strength associated
with a neurostimulation sequence currently being applied by the neurostimulation device, a somatic or psychologic state of the person (100), or an activity being carried out by
the person (100).

14. Neurostimulation device (105, 110) of any of claims 12 or 13, wherein the one or more second neurostimulation sequences comprises multi-phasic stimulation pulses, wherein each multi-phasic stimulation pulse comprises a stimulation phase and one or more active charge balancing phase; wherein the one or more active charge balancing phase of some or all of the multi-phasic stimulation pulses comprises a lower amplitude than the stimulation phase and a longer duration than the stimulation phase.

15. The neurostimulation device (105, 110) of any of claims 12 to 14, wherein each of the one or more second neurostimulation sequences comprises one or more of
an increased pulse frequency relative to the first neurostimulation sequence, an increased pulse amplitude relative to the first neurostimulation sequence or an increased pulse width relative to the first neurostimulation sequence,
such that a charge injected by each of the one or more second neurostimulation sequences is equal or larger than a charge injected by the first neurostimulation sequence; and / or
wherein the one or more second neurostimulation sequence was generated via execution of the computer program of any of claims 1 to 10.

## Patentansprüche

1. Computerprogramm zur Durchführung der folgenden Schritte, wenn es von einem Prozessor (220, 320) eines Neurostimulationssystems ausgeführt wird:
Anwenden einer Neurostimulationssequenz, die eine Vielzahl von Stimulationsimpulsen umfasst, unter Verwendung einer Neurostimulationsvorrichtung (105, 110) auf ein Nervensystem einer Person (100);
während
Bestimmen einer neurophysiologischen Wirkungsstärke, die durch die Neurostimulationssequenz verursacht wird, einer Nebenwirkungsstärke, die mit der Neurostimulationssequenz assoziiert ist, oder beidem; und
Erzeugen, basierend auf Bestimmen der neurophysiologischen Wirkungsstärke, der Nebenwirkungsstärke, oder beiden, einer modifizierten Neurostimulationssequenz, die eine erhöhte Ruhezeit aufweist;
wobei Erzeugen der modifizierten Neurostimulationssequenz umfasst:
Reduzieren einer Anzahl von Stimulationsimpulsen der Neurostimulationssequenz durch Einschließen einer oder mehrerer Ruheperioden in die Neurostimulationssequenz oder durch Entfernen von Stimulationsimpulsen aus der Neurostimulationssequenz oder
durch Durchführen beider; und
Erhöhen eines oder mehrerer von einer Impulsfrequenz, einer Impulsamplitude oder einer Impulsbreite der
Neurostimulationssequenz, um eine in das Nervensystem injizierte Ladung zu erhöhen;
Speichern der modifizierten Neurostimulationssequenz in einem nichtflüchtigen computerlesbaren Speichermedium; und, optional,
Anwenden der modifizierten Stimulationssequenz auf das Nervensystem der Person (100), um den neurophysiologischen Effekt zu bewirken.

2. Computerprogramm nach Anspruch 1, wobei Bestimmen der neurophysiologischen Wirkungsstärke umfasst Messen, über eine
Sensorvorrichtung, eines physiologischen Parameters der Person (100), der die neurophysiologische Wirkungsstärke angibt; und/oder
wobei der neurophysiologische Effekt eine Reduktion einer neurologischen Störung umfasst; und/oder
wobei Bestimmen der neurophysiologischen Wirkungsstärke Empfangen eines Rückkopplungssignals von der Person (100), das die neurophysiologische Wirkungsstärke angibt, umfasst; und/oder
wobei der neurophysiologische Effekt eine oder mehrere künstliche Empfindungen umfasst, die durch die Neurostimulationssequenz hervorgerufen werden.

3. Computerprogramm nach einem der Ansprüche 1 bis 2, wobei das Bestimmen der neurophysiologischen Wirkungsstärke eines oder mehrere der Folgenden umfasst:
Messen eines Parameters, der einen Grad der erfolgreichen Ausführung einer Verhaltensaufgabe (160) durch die Person (100) angibt, die durch den neurophysiologischen Effekt unterstützt wird, Messen der neuronalen Aktivität der Person (100), die mit dem neurophysiologischen Effekt assoziiert ist, Messen eines Parameters, der einen psychologischen Zustand der Person (100) angibt, der durch den neurophysiologischen Effekt beeinflusst wird, oder Bestimmen einer Leistungsmetrik einer Computer-Gehirn-Schnittstelle der Person (100) unter Verwendung des neurophysiologischen Effekts.

4. Computerprogramm nach einem der Ansprüche 1 bis 3, wobei Bestimmen der Nebenwirkungsstärke, die mit der Neurostimulationssequenz assoziiert ist, eines oder mehrere der Folgenden umfasst:
Messen eines Parameters, der einen Grad der erfolgreichen Ausführung einer Verhaltensaufgabe (160) durch die Person (100) angibt, der durch den Nebeneffekt beeinflusst wird, Messen der neuronalen Aktivität der Person (100), die mit dem Nebeneffekt assoziiert ist, Messen eines Parameters, der einen psychologischen Zustand der Person (100) angibt, der durch den Nebeneffekt beeinflusst wird, oder Bestimmen einer Leistungsmetrik einer Computer-Gehirn-Schnittstelle der Person (100), die durch den Nebeneffekt beeinflusst wird.

5. Computerprogramm nach einem der Ansprüche 1 bis 4, wobei Erzeugen der modifizierten Neurostimulationssequenz Folgendes umfasst:
Maximieren der Ruhezeit der Neurostimulationssequenz unter der Einschränkung, dass die bestimmte neurophysiologische Wirkungsstärke über einem vorkonfigurierten Schwellenwert für die neurophysiologische Wirkungsstärke bleibt; oder unter der Einschränkung, dass die bestimmte Nebenwirkungsstärke unter einem vorkonfigurierten Schwellenwert für die Nebenwirkungsstärke bleibt, oder beidem.

6. Computerprogramm nach Anspruch 5, wobei Erzeugen der modifizierten Neurostimulationssequenz Folgendes umfasst:
Reduzieren der Anzahl von Stimulationsimpulsen, bis die bestimmte neurophysiologische Wirkungsstärke unter den vorkonfigurierten Schwellenwert für die neurophysiologische Wirkungsstärke fällt, oder bis die bestimmte Nebenwirkungsstärke über den vorkonfigurierten Schwellenwert für die Nebenwirkungsstärke steigt, oder beidem; und
Erhöhen der durch die Neurostimulationssequenz in das Nervensystem injizierten Ladung bis die bestimmte neurophysiologische Wirkungsstärke über den vorkonfigurierten Schwellenwert für die neurophysiologische Wirkungsstärke steigt, oder bis die bestimmte Nebenwirkungsstärke unter den vorkonfigurierten Schwellenwert für die Nebenwirkungsstärke fällt, oder beidem.

7. Computerprogramm nach Anspruch 6, wobei die Anzahl von
Stimulationsimpulsen durch Aufnehmen einer oder mehrerer Ruheperioden zunehmender Länge in die Neurostimulationssequenz iterativ reduziert wird; und/oder
wobei die Anzahl von Stimulationsimpulsen durch zufälliges oder pseudozufälliges Entfernen von Stimulationsimpulsen aus der Neurostimulationssequenz basierend auf einer iterativ zunehmenden Stimulationsimpulsentfernungswahrscheinlichkeit pseudozufällig entfernt wird; und/oder
wobei Erzeugen der modifizierten Neurostimulationssequenz Wiederholen der Reduzierungs- und Erhöhungsschritte nach Anspruch 6 umfasst, bis die Ruhezeit der Neurostimulationssequenz über einem vorkonfigurierten Schwellenwert für die Ruhezeit liegt oder bis die Ruhezeit maximiert ist.

8. Computerprogramm nach den Ansprüchen 2 bis 7, wobei der Parameter, der die neurophysiologische Wirkungsstärke angibt, über eines oder mehrere der Folgenden gemessen wird:
einen Beschleunigungsmesser, der an einem Körperteil der Person (100) angebracht ist, ein Bildgebungssystem oder einen neuronalen Aktivitätssensor oder eine Verhaltensaufgabe; und
wobei der Parameter, der die neurophysiologische Wirkungsstärke angibt, vorzugsweise auf einer Leistungsspektraldichte eines Ausgangssignals von einem oder mehreren des Beschleunigungsmessers, des Bildgebungssystems oder des neuronalen Aktivitätssensors basiert.

9. Computerprogramm nach einem der vorhergehenden Ansprüche 1 bis 8, wobei Erzeugen der modifizierten Neurostimulationssequenz umfasst Modifizieren einer Impulsform von einem oder mehreren Stimulationsimpulsen der Neurostimulationssequenz; und/oder
wobei Erhöhen eines oder mehrerer von der Impulsfrequenz, der Impulsamplitude oder der Impulsbreite der Neurostimulationssequenz derart erfolgt, dass die durch die Neurostimulationssequenz in das Nervensystem injizierte Ladung aufrechterhalten oder erhöht wird.

10. Computerprogramm nach Anspruch 9, wobei die modifizierte Stimulationssequenz mehrphasige Stimulationsimpulse umfasst, wobei jeder mehrphasige Stimulationsimpuls eine Stimulationsphase und eine oder mehrere aktive Ladungsausgleichsphasen umfasst, und wobei Erzeugen der modifizierten Stimulationssequenz ferner Folgendes umfasst:
Modifizieren der Form der einen oder mehreren aktiven Ladungsausgleichsphase von einigen oder allen der mehrphasigen Stimulationsimpulse; und wobei vorzugsweise Modifizieren der Form der aktiven Ladungsausgleichsphase von einigen oder allen der mehrphasigen Stimulationsimpulse Folgendes umfasst:
Reduzieren einer Amplitude der aktiven Ladungsausgleichsphase und Erhöhen einer Dauer der aktiven Ladungsausgleichsphase.

11. System, umfassend:
eine Neurostimulationsvorrichtung (105, 110), die dazu ausgelegt ist, eine Vielzahl von Neurostimulationssequenzen, die jeweils eine Vielzahl von Stimulationsimpulsen umfassen, auf ein Nervensystem einer Person (100) anzuwenden; und
eine Programmiervorrichtung, die betriebsfähig mit der Neurostimulationsvorrichtung (105, 110) verbunden ist;
einen Speicher (240, 340), der das Computerprogramm nach einem der Ansprüche 1 bis 10 speichert, wobei die Programmiervorrichtung und die Neurostimulationsvorrichtung (105, 110) dazu ausgelegt sind, die Schritte des gespeicherten Computerprogramms durchzuführen.

12. Neurostimulationsvorrichtung (105, 110), umfassend:
einen Prozessor (220, 320), der betriebsfähig mit einer Neurostimulationsschaltung verbunden ist, die dazu ausgelegt ist, eine Vielzahl von Neurostimulationssequenzen zu erzeugen und auf ein Nervensystem einer Person (100) anzuwenden; und
einen Speicher (240, 340), der betriebsfähig mit dem Prozessor (220, 320) verbunden ist, der eine erste und eine oder mehrere zweite Neurostimulationssequenzen speichert, wobei jede Neurostimulationssequenz eine jeweilige Vielzahl von Stimulationsimpulsen umfasst und dazu ausgelegt ist, einen jeweiligen neurophysiologischen Effekt zu bewirken,
wobei die eine oder mehreren zweiten Neurostimulationssequenzen eine größere Ruhezeit als die erste Neurostimulationssequenz umfassen, indem sie weniger Stimulationsimpulse haben; und
wobei jede der einen oder der mehreren zweiten Neurostimulationssequenzen eines oder mehrere von einer erhöhten Impulsfrequenz relativ zu der ersten Neurostimulationssequenz, einer erhöhten Impulsamplitude relativ zu der ersten Neurostimulationssequenz oder einer erhöhten Impulsbreite relativ zu der ersten Neurostimulationssequenz umfasst.

13. Neurostimulationsvorrichtung (105, 110) nach Anspruch 12, wobei der Speicher (240, 340) eine Vielzahl von zweiten Neurostimulationssequenzen speichert, die jeweils eine unterschiedliche Ruhezeit und eine unterschiedliche erhöhte Impulsfrequenz, erhöhte Impulsamplitude und/oder erhöhte Impulsbreite aufweisen; und
wobei der Prozessor (220, 320) dazu ausgelegt ist, eine der Vielzahl von zweiten Neurostimulationssequenzen basierend auf einem Eingangssignal auszuwählen und anzuwenden; und
wobei das Eingangssignal optional eines oder mehrere von einer Benutzereingabe, einer zufällig erzeugten Auswahlanweisung oder einer Auswahlanweisung, die von einem vorkonfigurierten Therapieplan abgeleitet ist, umfasst; und
wobei das Eingangssignal optional ein Sensorsignal umfasst, das eines oder mehrere von Folgendem angibt: einer neurophysiologischen Wirkungsstärke der Person (100), einer Nebenwirkungsstärke, die mit einer Neurostimulationssequenz assoziiert ist, die aktuell von der Neurostimulationsvorrichtung angewendet wird, einem somatischen oder psychologischen Zustand der Person (100) oder einer Aktivität, die von der Person (100) durchgeführt wird.

14. Neurostimulationsvorrichtung (105, 110) nach einem der Ansprüche 12 oder 13, wobei die eine oder mehreren zweiten Neurostimulationssequenzen mehrphasige Stimulationsimpulse umfassen, wobei jeder mehrphasige Stimulationsimpuls eine Stimulationsphase und eine oder mehrere aktive Ladungsausgleichsphase umfasst;
wobei die eine oder die mehreren aktiven Ladungsausgleichsphase von einigen oder allen der mehrphasigen Stimulationsimpulse eine geringere Amplitude als die Stimulationsphase und eine längere Dauer als die Stimulationsphase umfasst.

15. Neurostimulationsvorrichtung (105, 110) nach einem der Ansprüche 12 bis 14, wobei jede der einen oder mehreren zweiten Neurostimulationssequenzen eines oder mehrere umfasst von einer erhöhten Impulsfrequenz relativ zu der ersten Neurostimulationssequenz, einer erhöhten Impulsamplitude relativ zu der ersten Neurostimulationssequenz oder eine erhöhten Impulsbreite relativ zu der ersten Neurostimulationssequenz, sodass eine durch jede der einen oder mehreren zweiten Neurostimulationssequenzen injizierte Ladung gleich oder größer als eine durch die erste Neurostimulationssequenz injizierte Ladung ist; und/oder wobei die eine oder mehreren zweiten Neurostimulationssequenzen durch Ausführung des Computerprogramms nach einem der Ansprüche 1 bis 10 erzeugt wurden.

## Revendications

1. Programme informatique pour l'exécution des étapes suivantes lorsqu'elles sont exécutées par un processeur (220, 320) d'un système de neurostimulation :
l'application à un système nerveux d'une personne (100), à l'aide d'un dispositif de neurostimulation (105, 110), d'une séquence de neurostimulation comprenant une pluralité d'impulsions de neurostimulation ;
ce faisant, la détermination d'une intensité d'un effet neurophysiologique produit par la séquence de neurostimulation, d'une intensité d'un effet secondaire associé à la séquence de neurostimulation, ou des deux ; et
la génération, sur la base de la détermination de l'intensité de l'effet neurophysiologique, de l'intensité de l'effet secondaire, ou des deux, d'une séquence de neurostimulation modifiée présentant un temps de quiescence accru, la génération de la séquence de neurostimulation modifiée comprenant :
la réduction d'un nombre d'impulsions de stimulation de la séquence de neurostimulation par inclusion d'une ou plusieurs périodes de quiescence dans la séquence de neurostimulation, ou par suppression d'impulsions de stimulation de la séquence de neurostimulation, ou en effectuant les deux ; et
l'augmentation d'une ou plusieurs d'entre une fréquence d'impulsions, une amplitude d'impulsion ou une largeur d'impulsion de la séquence de neurostimulation pour augmenter une charge injectée dans le système nerveux ;
le stockage de la séquence de neurostimulation modifiée dans un support de mémoire non transitoire lisible par calculateur ; et éventuellement
l'application de la séquence de neurostimulation modifiée au système nerveux de la personne (100) pour produire l'effet neurophysiologique.

2. Programme informatique de la revendication 1, dans lequel la détermination de l'intensité de l'effet neurophysiologique comprend la mesure, via un dispositif capteur, d'un paramètre physiologique de la personne (100) indiquant l'intensité de l'effet neurophysiologique ; et/ou dans lequel l'effet neurophysiologique comprend une réduction d'un trouble neurologique ; et/ou
dans lequel la détermination de l'intensité de l'effet neurophysiologique comprend la réception d'un signal en réaction provenant de la personne (100) indiquant l'intensité de l'effet neurophysiologique ; et/ou dans lequel l'effet neurophysiologique comprend une ou plusieurs sensations artificielles induites par la séquence de neurostimulation.

3. Programme informatique de l'une des revendications 1 à 2, dans lequel la détermination de l'intensité de l'effet neurophysiologique comprend une ou plusieurs d'entre :
la mesure d'un paramètre indiquant un degré d'exécution réussie d'une tâche comportementale (160) par la personne (100) aidée par l'effet neurophysiologique, la mesure d'une activité neuronale de la personne (100) associée à l'effet neurophysiologique, la mesure d'un paramètre indiquant un état psychologique de la personne (100) affectée par l'effet neurophysiologique, ou la détermination d'une métrique de performance d'une interface cerveau/calculateur de la personne (100) utilisant l'effet neurophysiologique.

4. Programme informatique de l'une des revendications 1 à 3, dans lequel la détermination de l'intensité de l'effet secondaire associé à la séquence de neurostimulation comprend une ou plusieurs d'entre :
la mesure d'un paramètre indiquant un degré d'exécution réussie d'une tâche comportementale (160) par la personne (100) affectée par l'effet secondaire, la mesure d'une activité neuronale de la personne (100) associée à l'effet secondaire, la mesure d'un paramètre indiquant un état psychologique de la personne (100) affectée par l'effet secondaire, ou la détermination d'une métrique de performance d'une interface cérébrale informatique de la personne (100) affectée par l'effet secondaire.

5. Programme informatique de l'une des revendications 1 à 4, dans lequel la génération de la séquence de neurostimulation modifiée comprend :
la maximisation du temps de quiescence de la séquence de neurostimulation avec la contrainte que l'intensité de l'effet neurophysiologique déterminée reste au-dessus d'un seuil préconfiguré pour l'intensité de l'effet neurophysiologique ; ou sous la contrainte que l'intensité de l'effet secondaire déterminée reste au-dessous d'un seuil préconfiguré pour l'intensité de l'effet secondaire ; ou les deux.

6. Programme informatique de la revendication 5, dans lequel la génération de la séquence de neurostimulation modifiée comprend :
la réduction du nombre d'impulsions de stimulation jusqu'à ce que l'intensité de l'effet neurophysiologique déterminée tombe au-dessous du seuil préconfiguré pour l'intensité de l'effet neurophysiologique, ou jusqu'à ce que l'intensité de l'effet secondaire déterminée augmente au-dessus du seuil préconfiguré pour l'intensité de l'effet secondaire, ou les deux ; et
l'augmentation de la charge injectée par la séquence de neurostimulation dans le système nerveux jusqu'à ce que l'intensité de l'effet neurophysiologique déterminée augmente au-dessus du seuil préconfiguré pour l'intensité de l'effet neurophysiologique, ou jusqu'à ce que l'intensité de l'effet secondaire déterminée tombe au-dessous du seuil préconfiguré pour l'intensité de l'effet secondaire, ou les deux.

7. Programme informatique de la revendication 6, dans lequel le nombre d'impulsions de stimulation est réduit de façon itérative par inclusion d'une ou plusieurs périodes de quiescence de longueur croissante dans la séquence de neurostimulation ; et/ou
dans lequel le nombre d'impulsions de stimulation est réduit de façon itérative par suppression aléatoire ou pseudo-aléatoire d'impulsions de stimulation de la séquence de neurostimulation sur la base d'une probabilité de suppression d'impulsions de stimulation itérativement croissante ; et/ou
dans lequel la génération de la séquence de neurostimulation modifiée comprend la répétition des étapes de réduction et d'augmentation de la revendication 6 jusqu'à ce que le temps de quiescence de la séquence de neurostimulation soit au-dessus d'un seuil préconfiguré pour le temps de quiescence ou jusqu'à ce que le temps de quiescence soit maximisé.

8. Programme informatique des revendications 2 à 7, dans lequel le paramètre indiquant l'intensité de l'effet neurophysiologique est mesuré via un ou plusieurs parmi : un accéléromètre fixé à une partie du corps de la personne (100), un système d'imagerie, ou un capteur d'activité neuronale, ou une tâche comportementale ; et de préférence dans lequel le paramètre indiquant l'intensité de l'effet neurophysiologique est basé sur une densité spectrale de puissance d'un signal de sortie d'un ou plusieurs parmi l'accéléromètre, le système d'imagerie, ou le capteur d'activité neuronale.

9. Programme informatique de l'une des revendications 1 à 8 précédentes, dans lequel la génération de la séquence de neurostimulation modifiée comprend la modification d'une forme d'impulsion d'une ou plusieurs impulsions de stimulation de la séquence de neurostimulation ; et/ou
dans lequel l'augmentation d'une ou plusieurs d'entre la fréquence d'impulsions, l'amplitude d'impulsion ou la largeur d'impulsion de la séquence de neurostimulation est effectuée de manière que la charge injectée par la séquence de neurostimulation modifiée dans le système nerveux soit maintenue ou accrue.

10. Programme informatique de la revendication 9, dans lequel la séquence de neurostimulation modifiée comprend des impulsions de stimulation multiphasiques, dans lequel chaque impulsion de stimulation multiphasique comprend une phase de stimulation et une ou plusieurs phases d'équilibrage actif de charge, et dans lequel la génération de la séquence de stimulation modifiée comprend en outre :
la modification de la forme des une ou plusieurs phases d'équilibrage actif de charge de certaines ou de toutes les impulsions de stimulation multiphasiques ; et de préférence dans lequel la modification de la forme de la phase d'équilibrage actif de charge de certaines ou de toutes les impulsions de stimulation multiphasiques comprend :
la réduction d'une amplitude de la charge d'équilibrage active de charge et l'augmentation d'une durée de la phase d'équilibrage actif de charge.

11. Système comprenant :
un dispositif de neurostimulation (105, 110) apte à appliquer à un système nerveux d'une personne (100) une pluralité de séquences de neurostimulation, comprenant chacune une pluralité d'impulsions de stimulation ; et
un dispositif de programmation, connecté de manière opérante au dispositif de neurostimulation (105, 110) ;
une mémoire (240, 340) stockant le programme informatique de l'une des revendications 1 à 10, le dispositif de programmation et le dispositif de neurostimulation (105, 110) étant aptes à exécuter les étapes du programme informatique stocké.

12. Dispositif de neurostimulation (105, 110) comprenant :
un processeur (220, 320) connecté de manière opérante à une circuiterie de neurostimulation apte à générer et à appliquer à un système nerveux d'une personne (100) une pluralité de séquences de neurostimulation ; et
une mémoire (240, 340) connectée de manière opérante au processeur (220, 320), stockant une première et une ou plusieurs secondes séquences de neurostimulation, chaque séquence de neurostimulation comprenant une pluralité respective d'impulsions de stimulation et étant apte à produire un effet neurophysiologique respectif,
dans lequel les une ou plusieurs secondes séquences de neurostimulation présentent un temps de quiescence supérieur à celui de la première séquence de neurostimulation, du fait qu'elles ont moins d'impulsions de stimulation ; et
dans lequel chacune des une ou plusieurs secondes séquences de neurostimulation présente une ou plusieurs parmi une fréquence d'impulsions accrue par rapport à la première séquence de neurostimulation, une amplitude d'impulsion accrue par rapport à la première séquence de neurostimulation, ou une largeur d'impulsion accrue par rapport à la première séquence de neurostimulation.

13. Dispositif de neurostimulation (105, 110) de la revendication 12, dans lequel la mémoire (240, 340) stocke une pluralité de secondes séquences de neurostimulation avec pour chacune un temps de quiescence différent et une fréquence d'impulsions accrue, une amplitude d'impulsion accrue et/ou une largeur d'impulsion accrue différentes ; et dans lequel le processeur (220, 320) est apte à sélectionner et à appliquer l'une de la pluralité de secondes séquences de neurostimulation sur la base d'un signal d'entrée ; et dans lequel, éventuellement, le signal d'entrée comprend une ou plusieurs d'entre une entrée utilisateur, une instruction de sélection générée aléatoirement, ou une instruction de sélection dérivée d'un protocole thérapeutique préconfiguré ; et dans lequel, éventuellement, le signal d'entrée comprend un signal de capteur représentatif d'une ou plusieurs d'entre : une intensité d'effet neurophysiologique de la personne, une intensité d'effet secondaire associée à une séquence de neurostimulation en cours d'application par le dispositif de neurostimulation, un état somatique ou psychologique de la personne (100), ou une activité qui est produite par la personne (100).

14. Dispositif de neurostimulation (105, 110) de l'une des revendications 12 ou 13, dans lequel les une ou plusieurs secondes séquences de neurostimulation comprennent des impulsions de stimulation multiphasiques, chaque impulsion de stimulation multiphasique comprenant une phase de stimulation et une ou plusieurs phases d'équilibrage actif de charge ; dans lequel les une ou plusieurs phases d'équilibrage actif de charge de certaines ou de toutes les impulsions de stimulation multiphasiques présentent une amplitude plus faible que celle de la phase de stimulation et une durée plus longue que celle de la phase de stimulation.

15. Le dispositif de neurostimulation (105, 110) de l'une des revendications 12 à 14, dans lequel chacune des une ou plusieurs secondes séquences de neurostimulation présente une ou plusieurs d'entre une fréquence d'impulsions accrue par rapport à celle de la première séquence de neurostimulation, une amplitude d'impulsion accrue par rapport à celle de la première séquence de neurostimulation, ou une largeur d'impulsion accrue par rapport à celle de la première séquence de neurostimulation, de sorte qu'une charge injectée par chacune des une ou plusieurs secondes séquences de neurostimulation soit égale ou supérieure à une charge injectée par la première séquence de neurostimulation ; et/ou
dans lequel les une ou plusieurs secondes séquences de neurostimulation avaient été générées par exécution du programme informatique de l'une des revendications 1 à 10.
